(19) **Europäisches Patentamt European Patent Office Office européen des brevets**

(11) **EP 4 027 183 B1**

(12) **EUROPEAN PATENT SPECIFICATION**

(45) Date of publication and mention of the grant of the patent:
**08.04.2026 Bulletin 2026/15**

(21) Application number: **20861636.7**

(22) Date of filing: **16.04.2020**

(51) International Patent Classification (IPC):
*G02B 23/24* (2006.01)       *A61B 1/00* (2006.01)
*A61B 1/045* (2006.01)      *A61B 5/06* (2006.01)
*A61B 5/00* (2006.01)       *A61B 1/05* (2006.01)
*A61B 1/07* (2006.01)

(52) Cooperative Patent Classification (CPC):
**A61B 1/00006; A61B 1/00009; A61B 1/00096; A61B 1/00158; A61B 1/05; A61B 1/07; A61B 5/062; A61B 5/4255;** A61B 5/004; A61B 5/063; A61B 5/7289; A61B 2562/0223

(86) International application number:
**PCT/JP2020/016696**

(87) International publication number:
**WO 2021/044661 (11.03.2021 Gazette 2021/10)**

(54) **ENDOSCOPIC SYSTEM AND METHOD FOR OPERATING THE SAME**

ENDOSKOPISCHES SYSTEM UND ENTSPRECHENDES VERFAHREN

SYSTÈME ENDOSCOPIQUE ET SON PROCÉDÉ DE FONCTIONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2019 JP 2019159793**

(43) Date of publication of application:
**13.07.2022 Bulletin 2022/28**

(73) Proprietor: **FUJIFILM Corporation Tokyo 106-8620 (JP)**

(72) Inventor: **YOKOTA, Tomonori Ashigarakami-gun, Kanagawa 258-8538 (JP)**

(74) Representative: **Dehns Germany Partnerschaft mbB Theresienstraße 6-8 80333 München (DE)**

(56) References cited:
EP-A1- 1 810 608          EP-A1- 2 105 085
EP-A1- 3 473 158          WO-A1-00/36427
WO-A1-2017/130352      WO-A1-2017/217162
JP-A- H1 176 154          JP-B2- 3 432 825

**Description**

**BACKGROUND OF THE INVENTION**

1. Field of the Invention

The present disclosure relates to an endoscope system and an operation method thereof.

2. Description of the Related Art

The technology of obtaining a position and an angle of a magnetic field detection element away from a magnetic field generation element based on the intensity of a magnetic field generated by the magnetic field generation element is disclosed (see JP3432825B). In this technology, a shape of an insertion part of the endoscope is obtained by obtaining the position and the angle of the magnetic field detection element provided in the insertion part of the endoscope. Monitoring an endoscope is disclosed in EP 3 473 158.

In general, the intensity of the magnetic field can be derived by normalizing a voltage of the electromotive force generated by the magnetic field detection element with a differential value of a current for driving the magnetic field generation element. The voltage of the electromotive force generated by the magnetic field detection element is input to a circuit network having an amplifier, a filter, and the like, and an output signal corresponding to the voltage of the electromotive force is output from the circuit network. The output signal of the circuit network is affected by a frequency characteristic of the circuit network.

In a case in which the output signal of the circuit network is normalized without considering this frequency characteristic, the intensity of the magnetic field may not be able to be derived with high accuracy. In this case, it is not possible to detect an insertion state including at least one of the position or the shape of the insertion part of the endoscope to be inserted into a subject with high accuracy. However, in the technology disclosed in JP3432825B, the frequency characteristic of the circuit network is not considered.

SUMMARY OF THE INVENTION

The present disclosure has been made in view of the above circumstances, and is to provide an endoscope system as defined by claim 1 and an operation method as defined by claim 5 thereof which can detect an insertion state including at least one of a position or a shape of an insertion part of an endoscope with high accuracy. The method of the claimed invention does not encompass any navigation or movement aspect or step.

An endoscope system according to the present disclosure comprises an acquisition unit that acquires a frequency characteristic of a circuit network, which is connected to a magnetic field detection element detecting a magnetic field and which outputs a detection signal corresponding to the magnetic field detected by the magnetic field detection element, a correction unit that generates a normalization waveform by correcting a differential waveform, which is obtained by differentiating a drive waveform of a magnetic field generation element generating the magnetic field, depending on the frequency characteristic, a derivation unit that derives intensity of the magnetic field by normalizing the detection signal output from the circuit network with the normalization waveform, and an insertion state detection unit that detects an insertion state including at least one of a position or a shape of an insertion part of an endoscope inserted into a subject by using the intensity of the magnetic field.

Note that in the endoscope system according to the present disclosure, the frequency characteristic may be stored in a storage unit provided in the endoscope.

In addition, the endoscope system according to the present disclosure may further comprise a second derivation unit that, by using a value relating to output impedance of the magnetic field detection element and a value relating to input impedance of a reception circuit provided in the circuit network and connected to the magnetic field detection element, derives a frequency characteristic of a pair of the magnetic field detection element and the reception circuit, in which the correction unit generates the normalization waveform by correcting the differential waveform depending on a second frequency characteristic derived by the second derivation unit in addition to a first frequency characteristic acquired by the acquisition unit.

In addition, in the endoscope system according to the present disclosure, the value relating to the output impedance may be a resistance value of the magnetic field detection element, and the value relating to the input impedance may be capacitance of a capacitor provided in a front stage of the reception circuit.

In addition, an operation method of an endoscope system according to the present disclosure comprises an acquisition step of acquiring a frequency characteristic of a circuit network, which is connected to a magnetic field detection element detecting a magnetic field and which outputs a detection signal corresponding to the magnetic field detected by the magnetic field detection element, a correction step of generating a normalization waveform by correcting a differential waveform, which is obtained by differentiating a drive waveform of a magnetic field generation element generating the magnetic field detected by the magnetic field detection element, depending on the frequency characteristic, a derivation step of deriving intensity of the magnetic field detected by the magnetic field detection element by normalizing the detection signal output from the circuit network with the normalization waveform, and an insertion state detection step of detecting an insertion state including at least one of a position or a shape of an insertion part of an endoscope inserted into a subject by using the intensity of the magnetic field.

According to the present disclosure, it is possible to detect the insertion state including at least one of the position or the shape of the insertion part of the endoscope with high accuracy.

## BRIEF DESCRIPTION OF THE DRAWINGS

Fig. 1 is an explanatory diagram showing a state of an endoscopic examination using an endoscope system.

Fig. 2 is a schematic view showing an overall configuration of the endoscope system.

Fig. 3 is a block diagram showing an electric configuration of the endoscope system according to a first embodiment.

Fig. 4 is a block diagram showing an electric configuration of a magnetic field detection circuit.

Fig. 5 is an explanatory diagram showing a state in which a plurality of detection coils detect a magnetic field generated by a plurality of generation coils.

Fig. 6 is an explanatory diagram showing an example of magnetic field measurement data.

Fig. 7 is a diagram for describing a determination process by a determination unit and a position detection process of each detection coil by a position detection unit.

Fig. 8 is a diagram for describing a control of switching the plurality of generation coils and a control of switching the plurality of detection coils.

Fig. 9 is a diagram for describing a derivation process the intensity of the magnetic field.

Fig. 10 is a diagram showing an example of a frequency characteristic table.

Fig. 11 is a diagram showing an example of a gain stored as a frequency characteristic in the frequency characteristic table according to each embodiment.

Fig. 12 is a diagram showing an example of a phase stored as the frequency characteristic in the frequency characteristic table according to each embodiment.

Fig. 13 is a diagram showing an example of the gain stored as the frequency characteristic in the frequency characteristic table according to a modification example.

Fig. 14 is a diagram showing an example of the phase stored as the frequency characteristic in the frequency characteristic table according to the modification example.

Fig. 15 is a block diagram showing an example of a functional configuration of an insertion state detection unit according to the invention.

Fig. 16 is a diagram for describing a correction process of a differential waveform based on the frequency characteristics according to the invention.

Fig. 17 is an explanatory diagram showing an example of a shape detection process of an insertion part.

Fig. 18 is a flowchart showing an example of a display process of an observation image and an insertion part shape image according to each embodiment.

Fig. 19 is a block diagram showing an electric configuration of the endoscope system according to a second embodiment.

Fig. 20 is a diagram showing an example of a circuit configuration of an equivalent circuit of the detection coil and an instrumentation amplifier.

Fig. 21 is a diagram showing an example of a resistance value table.

Fig. 22 is a diagram showing an example of a capacitance table.

Fig. 23 is a block diagram showing an example of a functional configuration of the insertion state detection unit according to the second embodiment.

Fig. 24 is a diagram for describing an installation position and an installation angle of the generation coil according to the modification example.

Fig. 25 is a diagram for describing an actual installation position of the generation coil according to the modification example.

Fig. 26 is a diagram for describing an actual installation angle of the generation coil according to the modification example.

## DESCRIPTION OF THE PREFERRED EMBODIMENTS

Hereinafter, an embodiment for carrying out the technology of the present disclosure will be described in detail with reference to the drawings.

[First Embodiment]

<Overall Configuration of Endoscope System>

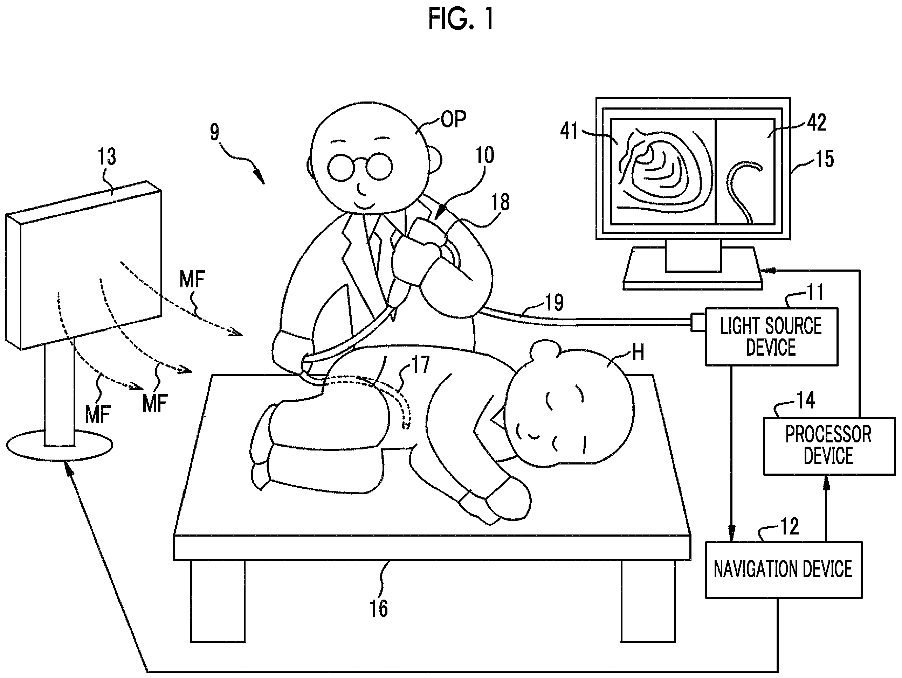

Fig. 1 is a schematic view showing an overall configuration of an endoscope system 9 according to the technology of the present disclosure. As shown in Fig. 1, the endoscope system 9 comprises an endoscope 10, a light source device 11, a navigation device 12, a magnetic field generator 13, a processor device 14, and a monitor 15. The endoscope system 9 is used for an endoscopic examination in a body of a subject H, such as a patient. The subject H is an example of a subject. The endoscope 10 is, for example, an endoscope inserted into the digestive tract, such as the large intestine, and is a soft endoscope having flexibility. The endoscope 10 has an insertion part 17 to be inserted into the digestive tract, an operating part 18 installed consecutively to a proximal end side of the insertion part 17 and gripped by an operator OP to perform various operations, and a universal cord 19 installed consecutively to the operating part 18.

The endoscopic examination is performed, for example, in a state in which the subject H lies on a bed 16. In a case of a large intestine examination, the insertion part 17 of the endoscope 10 is inserted into the digestive tract from an anus by an operator OP who is the doctor. The light source device 11 supplies illumination light that illuminates an inside of the large intestine, which is an observation site, to the endoscope 10. The processor device 14 generates an observation image 41 by processing an image captured by the endoscope 10. The observation image 41 is displayed on the monitor 15. The operator OP proceeds with the endoscopic examination while confirming the observation image 41. The observation image 41 displayed on the monitor 15 is basically a motion picture, but it is also possible to display a still picture as the observation image 41 as needed.

In addition, the endoscope system 9 has a navigation function of navigating technique, such as an insertion operation of the endoscope 10 performed by the operator OP. Here, the navigation means supporting the technique of the endoscope 10 of the operator OP by presenting an insertion state including at least one of the position or the shape of the insertion part 17 of the endoscope 10 in the body of the subject H to the operator OP. The navigation function is a function of presenting the insertion state of the insertion part 17 is detected using a magnetic field MF and the detected insertion state. In the present embodiment, the insertion state includes a position of a part of the insertion part 17 inserted into the body (for example, a distal end part 23 described below), as well as the shape of the entire insertion part 17 in the body.

The navigation function is realized by the navigation device 12, the magnetic field generator 13, and a magnetic field measurement device in the endoscope 10 described below. The magnetic field generator 13 generates the magnetic field MF. The magnetic field generator 13 is attached to, for example, a stand and is disposed beside the bed 16 on which the subject H lies. In addition, the magnetic field generator 13 is disposed within a range in which the generated magnetic field MF reaches the body of the subject H.

The magnetic field measurement device in the endoscope 10 detects the magnetic field MF generated by the magnetic field generator 13 and measures the intensity of the detected magnetic field MF. The navigation device 12 detects the insertion state of the insertion part 17 by deriving a relative position between the magnetic field generator 13 and the insertion part 17 based on a magnetic field measurement result by the magnetic field measurement device. The processor device 14 generates an insertion part shape image 42, which shows the insertion state detected by the navigation device 12.

The monitor 15 displays the observation image 41 and the insertion part shape image 42. Note that the monitor 15 that displays the observation image 41 and the insertion part shape image 42 may be provided separately.

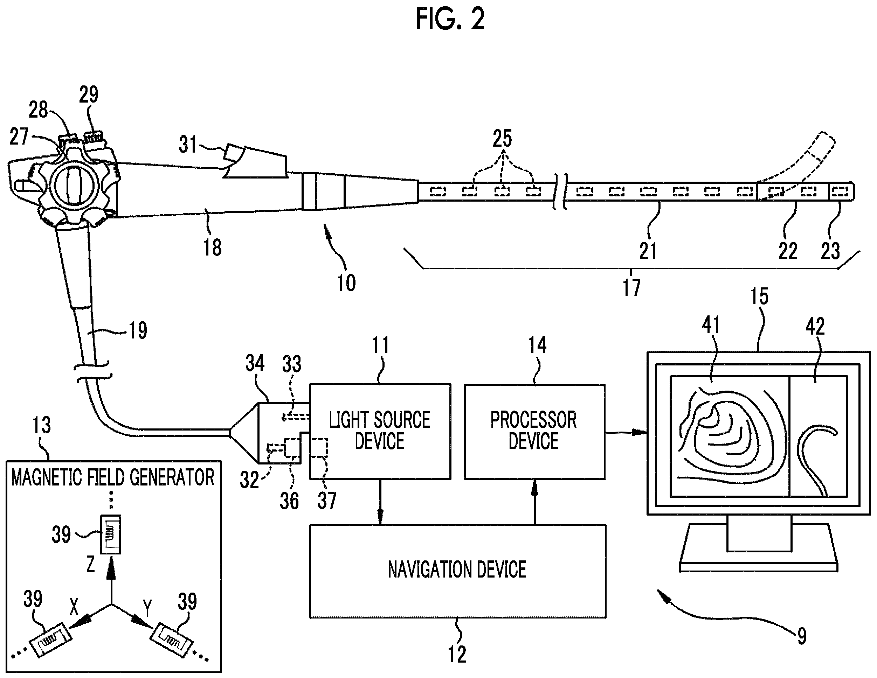

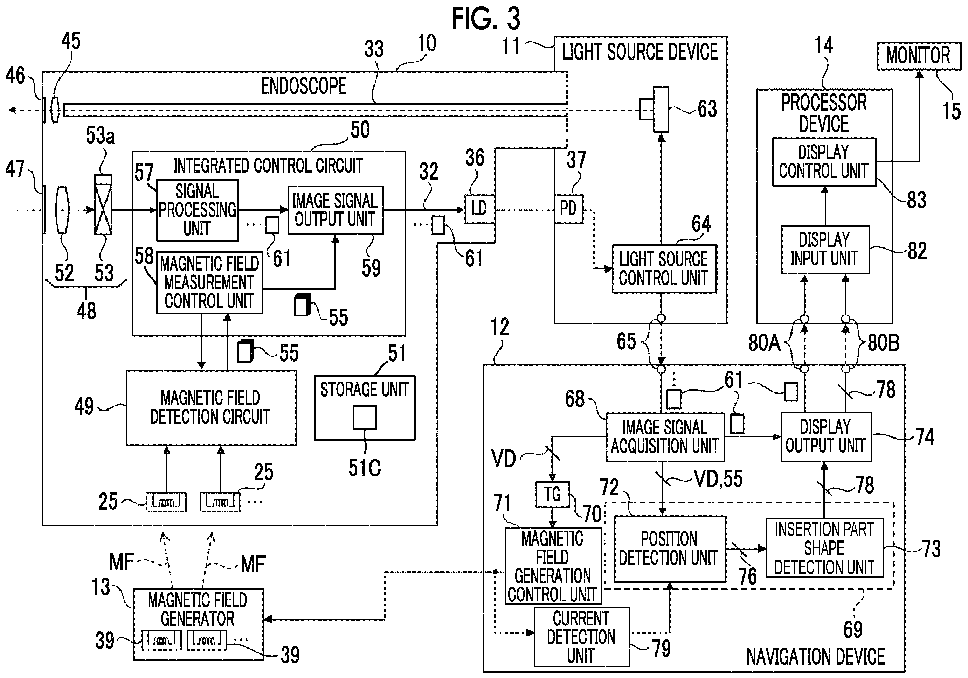

As shown in Fig. 2, the insertion part 17 is a tubular portion having a small diameter and a long length, and is configured by a soft part 21, a bendable part 22, and a distal end part 23, which are connected sequentially from the proximal end side to the distal end side. The soft part 21 has flexibility. The bendable part 22 is a part that can be bent by the operation of the operating part 18. An imaging apparatus 48 (see Fig. 3) and the like are disposed at the distal end part 23. In addition, although not shown, an illumination window 46 (see Fig. 3) that illuminates the observation site with the illumination light, an observation window 47 (see Fig. 3) on which subject light of the illumination light reflected by the subject is incident, a treatment tool outlet for a treatment tool to protrude, and a cleaning nozzle for cleaning the observation window 47 by injecting gas and water into the observation window 47 are provided on a distal end surface of the distal end part 23.

A light guide 33, a signal cable 32, an operation wire (not shown), and a pipe line for inserting the treatment tool (not shown) are provided in the insertion part 17. The light guide 33 extends from the universal cord 19 and guides the illumination light supplied from the light source device 11 to the illumination window 46 at the distal end part 23. The signal cable 32 is used for power supply to the imaging apparatus 48 in addition to communication of an image signal from the imaging apparatus 48 and a control signal for controlling the imaging apparatus 48. Like the light guide 33, the signal cable 32 also extends from the universal cord 19 and is arranged to the distal end part 23.

The operation wire is a wire for operating the bendable part 22, and is arranged from the operating part 18 to the bendable part 22. The pipe line for inserting the treatment tool is a pipe line for inserting the treatment tool (not shown), such as forceps, and is arranged from the operating part 18 to the distal end part 23. In addition, a fluid tube for air/water supply is provided in the insertion part 17. The fluid tube supplies the distal end part 23 with the gas and the water for cleaning the observation window 47.

In addition, in the insertion part 17, a plurality of detection coils 25 are provided from the soft part 21 thereof to the distal end part 23 at preset intervals. Each detection coil 25 corresponds to a magnetic field detection element which detects the magnetic field MF. Each detection coil 25 is affected by the magnetic field MF generated from the magnetic field generator 13, so that an induced electromotive force is generated by an operation of electromagnetic induction, and an induced current is generated by the induced electromotive force. A value of the induced current generated from each detection coil 25 is a value corresponding to the intensity of the magnetic field MF detected by each detection coil 25, which is the magnetic field measurement result. That is, the magnetic field measurement result refers to a value depending on the magnitude of the induced current.

The operating part 18 is provided with various operation members operated by the operator OP. Specifically, the operating part 18 is provided with two types of bending operation knobs 27, an air/water supply button 28, and a suction button 29. Each of the two types of bending operation knobs 27 is connected to the operation wire, and is used for a right-left bending operation and an up-down bending operation of the bendable part 22. In addition, the operating part 18 is provided with a treatment tool inlet port 31 which is an inlet of the pipe line for inserting the treatment tool.

The universal cord 19 is a connection cord that connects the endoscope 10 to the light source device 11. The universal cord 19 encompasses the signal cable 32, the light guide 33, and the fluid tube (not shown). In addition, a connector 34 connected to the light source device 11 is provided at an end part of the universal cord 19.

By connecting the connector 34 to the light source device 11, the light source device 11 supplies the power, the control signal, the illumination light, the gas, and the water necessary for operating the endoscope 10 to the endoscope 10. In addition, the image signal of the observation site acquired by the imaging apparatus 48 (see Fig. 3) of the distal end part 23 and the magnetic field measurement result based on a detection signal of each detection coil 25 are transmitted from the endoscope 10 to the light source device 11.

The connector 34 is not electrically connected to the light source device 11 by wire using a metal signal line or the like, and instead, the connector 34 and the light source device 11 are connected to each other so as to be capable of optical communication (non-contact type communication). The connector 34 transmits and receives the control signal exchanged between the endoscope 10 and the light source device 11 and transmits the image signal and the magnetic field measurement result from the endoscope 10 to the light source device 11 by optical communication. The connector 34 is provided with a laser diode (hereinafter, referred to as "LD") 36 connected to the signal cable 32.

The LD 36 is used for transmitting a large amount of data from the endoscope 10 to the light source device 11, specifically, transmitting the image signal and the magnetic field measurement result. The LD 36 transmits the image signal and the magnetic field measurement result, which has been originally in a form of the electric signal, in a form of an optical signal to a photodiode (hereinafter, referred to as "PD") 37 provided in the light source device 11.

Note that, although not shown, apart from the LD 36 and the PD 37, both the connector 34 and the light source device 11 are provided with a light transmission and reception unit that converts a small amount of the control signal exchanged between the endoscope 10 and the light source device 11 into the optical signal and transmits and receives the converted optical signal. Further, the connector 34 is provided with a power reception unit (not shown) that receives the power supplied from a power feed unit (not shown) of the light source device 11 by wireless power supply.

The light guide 33 in the connector 34 is inserted into the light source device 11. In addition, the fluid tube (not shown) in the connector 34 is connected to an air/water supply device (not shown) via the light source device 11. As a result, the light source device 11 and the air/water supply device supply the illumination light, the gas, and the water to the endoscope 10, respectively.

The light source device 11 supplies the illumination light to the light guide 33 of the endoscope 10 via the connector 34, and supplies the gas and the water supplied from the air/water supply device to the fluid tube of the endoscope 10. In addition, the light source device 11 receives the optical signal transmitted from the LD 36 by the PD 37, converts the received optical signal into the original image signal, which is the electric signal, and the magnetic field measurement result, and then outputs the converted image signal and the magnetic field measurement result to the navigation device 12.

The navigation device 12 outputs the image signal for generating the observation image 41 input from the light source device 11 to the processor device 14. In addition, the navigation device 12 controls the drive of the magnetic field generator 13 described below, detects the insertion state of the insertion part 17 in the body of the subject H, and outputs a detection result to the processor device 14 as the information for generating the insertion part shape image 42.

As described above, the endoscope 10 in the present embodiment is one-connector type having one connector 34 connected to the light source device 11. The endoscope 10 is connected to each of the processor device 14 and the navigation device 12 so as to be capable of communication via the light source device 11 to which the connector 34 is connected.

The magnetic field generator 13 has a plurality of generation coils 39 corresponding to a plurality of magnetic field generation elements. Each generation coil 39 includes, for example, an X-axis coil, a Y-axis coil, and a Z-axis coil that generate, by applying a drive current, an alternating current magnetic field in directions corresponding to XYZ coordinate axes of an orthogonal coordinate system XYZ, respectively. Each generation coil 39 generates the magnetic field MF having the same frequency. The generation coils 39 generate the magnetic fields MF at different timings from each other under a control of the navigation device 12, which will be described in detail below.

<Endoscope>

Fig. 3 is a block diagram showing an electric configuration of the endoscope system 9. As shown in Fig. 3, the endoscope 10 includes the light guide 33, an irradiation lens 45, the illumination window 46, the observation window 47, the imaging apparatus 48, a magnetic field detection circuit 49, an integrated control circuit 50, a storage unit 51, the signal cable 32, the LD 36, and the fluid tube and the cleaning nozzle (which are not shown).

The light guide 33 is, for example, a large-diameter optical fiber or a bundle fiber. An incident end of the light guide 33 is inserted into the light source device 11 via the connector 34. The light guide 33 is inserted into the connector 34, the universal cord 19, and the operating part 18, and the insertion part 17, and an emission end faces the irradiation lens 45 provided in the distal end part 23 of the insertion part 17. As a result, the illumination light supplied from the light source device 11 to the incident end of the light guide 33 is emitted to the observation site from the irradiation lens 45 through the illumination window 46 provided on the distal end surface of the distal end part 23. Moreover, the illumination light reflected by the observation site is incident on an imaging surface of the imaging apparatus 48 as image light of the observation site through the observation window 47 provided on the distal end surface of the distal end part 23.

Note that one end side of the fluid tube described above is connected to the air/water supply device through the connector 34 and the light source device 11, and the other end side of the fluid tube is connected to an air/water supply nozzle (not shown) provided on the distal end surface of the distal end part 23 through the insertion part 17 and the like. As a result, the gas or the water supplied from the air/water supply device is injected into the observation window 47 from the air/water supply nozzle to clean the observation window 47.

The imaging apparatus 48 includes a condenser lens 52 and an imaging element 53. The condenser lens 52 collects the image light of the observation site incident from the observation window 47, and forms the collected image light of the observation site on the imaging surface of the imaging element 53. The imaging element 53 is a complementary metal oxide semiconductor (CMOS) type or charge coupled device (CCD) type imaging element. The imaging element 53 is, for example, a color imaging element in which any of R, G, or B microfilter is assigned to each pixel. The imaging element 53 images the observation site, which is an observation target. More specifically, the imaging element 53 images the image light of the observation site imaged on the imaging surface (converts the image light into the electric signal), and outputs the image signal of the observation site to the integrated control circuit 50.

In addition, the imaging element 53 is provided with an oscillation unit 53a that outputs a reference signal (clock signal), such as a crystal oscillator, and the imaging element 53 outputs the image signal constituting the motion picture with the reference signal oscillated from the oscillation unit 53a as a reference. An interval of the reference signal defines a frame rate. The frame rate is, for example, 30 frames per second (fps).

The magnetic field detection circuit 49 is electrically connected to each detection coil 25 in the insertion part 17. The magnetic field detection circuit 49 outputs magnetic field measurement data 55 including the magnetic field measurement result of each detection coil 25 depending on the magnetic field MF generated from the generation coil 39 of the magnetic field generator 13 to the integrated control circuit 50.

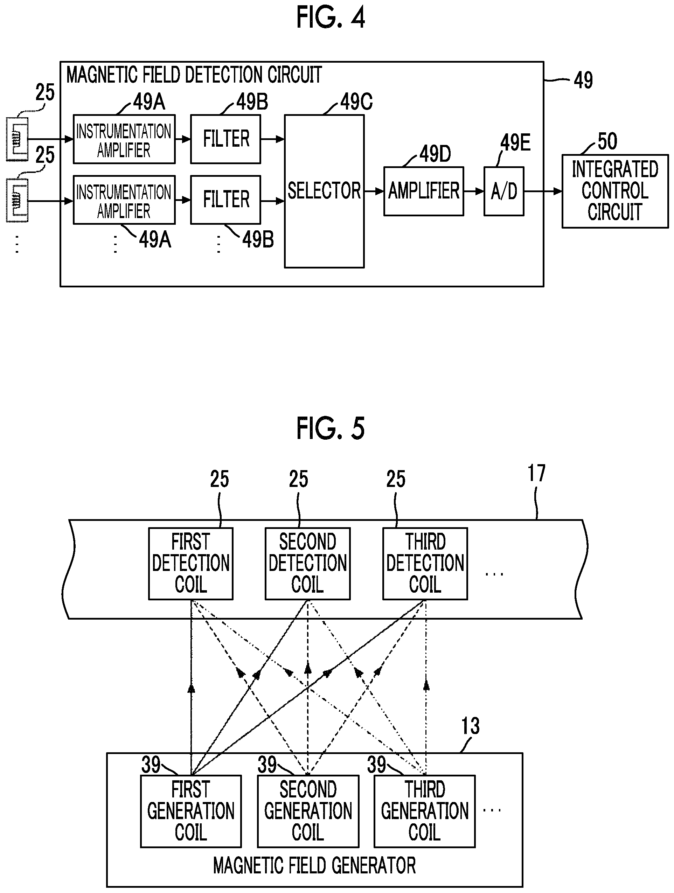

Specifically, as shown in Fig. 4, the magnetic field detection circuit 49 includes an instrumentation amplifier 49A, a filter 49B, a selector 49C, an amplifier 49D, and an analog/digital (A/D) conversion circuit 49E. The instrumentation amplifier 49A is provided for each detection coil 25, and an input terminal thereof is connected to each detection coil 25. The instrumentation amplifier 49A amplifies a weak detection signal (induced current) output by each detection coil 25 and outputs the amplified detection signal as a voltage corresponding to the value of the detection signal. An output terminal of each instrumentation amplifier 49A is connected to the filter 49B. The instrumentation amplifier 49A is an example of a reception circuit that receives the signal output from the detection coil 25. In addition, a pair of the instrumentation amplifier 49A and the filter 49B is an example of a circuit network that outputs the detection signal corresponding to the magnetic field detected by the detection coil 25.

The filter 49B is a filter, such as a low pass filter (LPF) or a band pass filter (BPF), and is provided for each instrumentation amplifier 49A, and an input terminal thereof is connected to each instrumentation amplifier 49A. The filter 49B passes a signal of a preset frequency band among the signals input from the instrumentation amplifier 49A. An output terminal of each filter 49B is connected to the selector 49C.

The selector 49C selects the filter 49B, which is a readout target, from the filters 49B. Among a plurality of the filters 49B, the voltage from the filter 49B selected by the selector 49C is input to the amplifier 49D via the selector 49C. The selector 49C sequentially selects each filter 49B based on a timing signal input from a magnetic field measurement control unit 58. Therefore, the voltages in response to the detection signals of the plurality of detection coils 25 are sequentially read out.

The amplifier 49D amplifies the voltage output from the selector 49C. More specifically, the amplifier 49D is, for example, an operational amplifier, and amplifies a voltage value of a difference between an input voltage input from the selector 49C and a reference voltage input as a reference and output the amplified voltage value. The A/D conversion circuit 49E converts an output voltage of the amplifier 49D into a digital signal. The A/D conversion circuit 49E outputs a value of this digital signal as the magnetic field measurement result of each detection coil 25 (value corresponding to the magnitude of the induced current indicating the intensity of the magnetic field MF). By sequentially selecting each detection coil 25 by the selector 49C, the A/D conversion circuit 49E outputs the magnetic field measurement data 55 including the respective magnetic field measurement results based on the detection signals of all of the detection coils 25 to the integrated control circuit 50.

The integrated control circuit 50 configured to include arithmetic circuits including various central processing units (CPU) and various memories, and controls the operations of the units of the endoscope 10 in an integrated manner. The integrated control circuit 50 functions as a signal processing unit 57, a magnetic field measurement control unit 58, and an image signal output unit 59 by executing a control program stored in a memory (not shown). The magnetic field detection circuit 49 and the magnetic field measurement control unit 58 correspond to a magnetic field measurement unit. The magnetic field measurement unit measures a plurality of the magnetic fields MF originating from the generation coils 39 corresponding to the plurality of magnetic field generation elements based on the detection signals output by the detection coils 25, and outputs the magnetic field measurement result for each magnetic field MF. The magnetic field measurement unit and the detection coil 25 are combined to configure the magnetic field measurement device.

The signal processing unit 57 performs various signal processes on the image signals sequentially output from the imaging element 53. The signal process includes, for example, an analog signal process, such as a sampling two correlation pile process and a signal amplification process, and an A/D conversion process of converting an analog signal into a digital signal after the analog signal process. The image signal after the signal process is performed is called a frame image signal 61. The signal processing unit 57 outputs the frame image signal 61 to the image signal output unit 59 depending on the frame rate. The frame image signal 61 is used as motion picture data of the observation site. As described above, a plurality of the frame image signals 61 are the image signals that are acquired by the imaging element 53 executing motion picture imaging and are output at time intervals depending on the frame rate.

The magnetic field measurement control unit 58 acquires the magnetic field measurement data 55 including a plurality of the magnetic field measurement results of the detection coils 25 via the magnetic field detection circuit 49, and outputs the acquired magnetic field measurement data 55 to the image signal output unit 59.

As shown in Fig. 5, even in a case in which the intensity of the magnetic field generated by each generation coil 39 is the same, for example, the magnetic field measurement result of each detection coil 25 is changed depending on a distance and a direction between each generation coil 39 that generates the magnetic field MF and each detection coil 25. For example, the first generation coil 39 shown in Fig. 5 has different distance and direction from each of the first to third detection coils 25, as shown by a solid line. Therefore, the magnetic field measurement results of the first to third detection coils 25 for the magnetic field MF generated by one first generation coil 39 are different. A relationship between each of the second generation coil 39 and the third generation coil 39 and each of the first to third detection coils 25 is the same.

In addition, on the contrary, even in a case in which the intensity of the magnetic field MF generated by each of the first to third generation coils 39 is the same, the magnetic field measurement result of one first detection coil 25 for the magnetic field MF of each generation coil 39 is different. Here, for example, a case is considered in which the first to third generation coils 39 are the X-axis coil, the Y-axis coil, and the Z-axis coil, respectively. In this case, a three-dimension coordinate position of the first detection coil 25 corresponding to the XYZ coordinate axes can be detected based on the magnetic field measurement result of one first detection coil 25 for the magnetic field MF of each of the X-axis coil, the Y-axis coil, and the Z-axis coil. The same applies to the second detection coil 25 and the third detection coil 25. In a case in which the three-dimension coordinate positions of the detection coils 25 provided in the insertion part 17 at preset intervals can be detected, the shape of the insertion part 17 can be detected. In addition, in a case in which the three-dimension coordinate positions of the detection coils 25 disposed in the vicinity of the distal end part 23 can be detected, the position of the distal end part 23 can be detected.

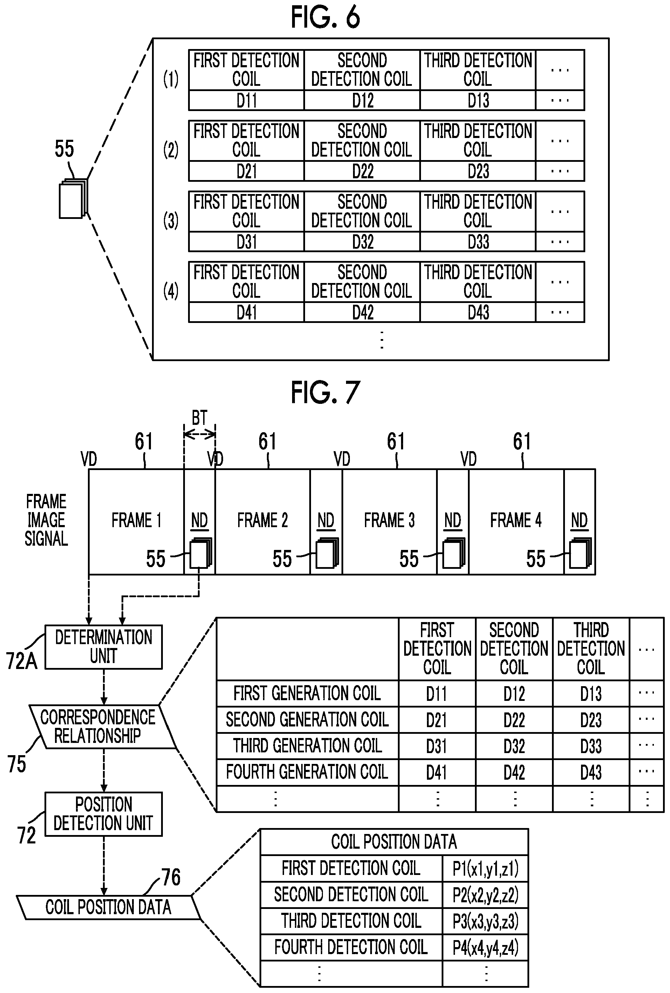

Fig. 6 is an explanatory diagram for describing an example of the magnetic field measurement data 55 acquired by the magnetic field measurement control unit 58. The magnetic field measurement control unit 58 detects the plurality of magnetic fields MF generated by the plurality of generation coils 39 by the plurality of detection coils 25 and acquires the magnetic field measurement data 55 including the plurality of magnetic field measurement results output from the detection coils 25.

In Fig. 6, (1) to (4) are data strings showing the magnetic field measurement results of the plurality of detection coils 25 with respect to the magnetic fields MF generated by the generation coils 39, respectively. For example, "D11" is a magnetic field measurement result in which the magnetic field MF generated by the first driven generation coil 39 is detected by the "first detection coil". "D12" is a magnetic field measurement result in which the magnetic field MF generated by the first driven generation coil 39 is detected by the "second detection coil". Similarly, "D42" is a magnetic field measurement result in which the magnetic field MF generated by the fourth driven generation coil 39 is detected by the "second detection coil". "D43" is a magnetic field measurement result in which the magnetic field MF generated by the fourth driven generation coil 39 is detected by the "third detection coil".

The magnetic field measurement control unit 58 sequentially acquires the magnetic field measurement results of the detection coils 25 while synchronizing with a magnetic field generation timing of each generation coil 39 in the magnetic field generator 13. The magnetic field measurement control unit 58 acquires, for example, the magnetic field measurement results of all of the detection coils 25 for the magnetic fields MF of all of the generation coils 39 in one magnetic field measurement period defined by a synchronization signal described below. As a result, the magnetic field measurement control unit 58 acquires the magnetic field measurement data 55 including the plurality of magnetic field measurement results relating to all of combinations of the generation coils 39 and the detection coils 25 in one magnetic field measurement period. The magnetic field measurement period corresponds to a predetermined measurement unit time of the magnetic field. In the present embodiment, the magnetic field measurement period is an interval of the reference signal that defines the frame rate of the imaging element 53.

For example, in a case in which 9 generation coils 39 are provided in the magnetic field generator 13 and 17 detection coils 25 are provided in the insertion part 17, 17 magnetic field measurement results can be obtained for each generation coil 39. Therefore, the magnetic field measurement control unit 58 acquires the magnetic field measurement data 55 including a total of $9 \times 17 = 153$ magnetic field measurement results in one magnetic field measurement period. The magnetic field measurement data 55 including the plurality of magnetic field measurement results relating to all of such combinations is referred to as total magnetic field measurement data. In the present embodiment, unless otherwise specified, the magnetic field measurement data 55 shall include the total magnetic field measurement data.

As shown in Fig. 7, the image signal output unit 59 adds a frame start signal VD to each of the plurality of frame image signals 61 sequentially input from the signal processing unit 57, and outputs the frame image signal 61. In Fig. 7, "frame 1", "frame 2", "frame 3" ... are frame numbers indicating an output order of the plurality of frame image signals 61, which are shown for convenience. The frame start signal VD is a vertical synchronization signal, for example.

Further, the image signal output unit 59 adds the magnetic field measurement data 55 to the frame image signal 61, and outputs the frame image signal 61. That is, the frame start signal VD and the magnetic field measurement data 55 are included in all of the frame image signals 61 output by the image signal output unit 59. As shown in Fig. 7, the magnetic field measurement data 55 is added to a signal invalid region ND between the frame image signals 61, which corresponds to a blanking time BT of the imaging element 53. The blanking time BT is a vertical blanking period, for example. The frame start signal VD is also the signal included in the vertical blanking period of the plurality of frame image signals 61.

Via the signal cable 32, the image signal output unit 59 outputs the frame image signal 61 to the LD 36. The LD 36 transmits the optical signal obtained by converting the frame image signal 61 into the optical signal to the PD 37 of the light source device 11.

As described above, the image signal output unit 59 outputs the frame image signal 61 acquired from the imaging element 53 via the signal processing unit 57 to the outside of the endoscope 10. In addition, by using the frame start signal VD included in the frame image signal 61 as the synchronization signal, the image signal output unit 59 functions as a synchronization signal generation unit.

<Light Source Device>

The light source device 11 includes an illumination light source 63, the PD 37, a light source control unit 64, and a communication interface 65. The illumination light source 63 is, for example, a semiconductor light source, such as the LD or a light emitting diode (LED), and is a white light source which emits white light having a wavelength range from a red region to a blue region as the illumination light. Note that, as the illumination light source 63, in addition to the white light source, a special light source that emits special light, such as purple light and infrared light, may be used. The illumination light emitted from the illumination light source 63 is incident on the incident end of the light guide 33.

The PD 37 receives the optical signal transmitted from the LD 36. The PD 37 converts the frame image signal 61 received in the form of an optical signal into the form of the original electric signal. The converted frame image signal 61 from the PD 37 is input to the light source control unit 64.

The light source control unit 64 is configured to include various arithmetic circuits including the CPU and various memories, and controls the operation of each unit of the light source device 11, such as the illumination light source 63. In addition, the converted frame image signal 61 from the PD 37 is output to the navigation device 12 via the light source control unit 64 and the communication interface 65.

<Navigation Device>

The navigation device 12 includes an image signal acquisition unit 68, an insertion state detection unit 69, a timing generator (hereinafter referred to as "TG") 70, a magnetic field generation control unit 71, a display output unit 74, and a current detection unit 79. Each unit of the navigation device 12 is configured by various arithmetic circuits (not shown) including one or a plurality of CPUs, and is operated by executing a control program stored in a memory (not shown).

Via the communication interface 65, the image signal acquisition unit 68 acquires the frame image signal 61 from the light source control unit 64. Moreover, the image signal acquisition unit 68 outputs the acquired frame image signal 61 to the display output unit 74.

In addition, the image signal acquisition unit 68 extracts the frame start signal VD and the magnetic field measurement data 55 included in the frame image signal 61, and outputs the extracted frame start signal VD and the magnetic field measurement data 55 to the insertion state detection unit 69. In addition, the image signal acquisition unit 68 outputs the frame start signal VD extracted from the frame image signal 61 to the TG 70.

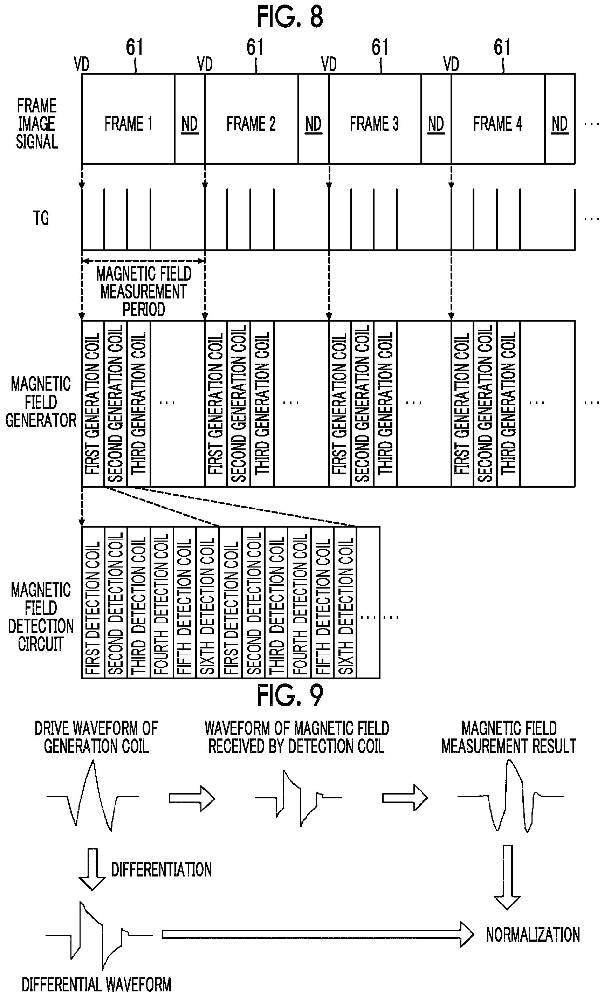

Based on the frame start signal VD input from the image signal acquisition unit 68, the TG 70 outputs a clock signal for control of selectively switching each generation coil 39 to the magnetic field generation control unit 71. As shown in Fig. 8 as an example, the magnetic field generation control unit 71 performs a control of driving each of the plurality of generation coils 39 while shifting a drive timing based on the clock signal input from the TG 70.

As shown in Fig. 8, each magnetic field measurement period is based on the clock signal input from the TG 70 to the magnetic field generation control unit 71, which corresponds to the frame start signal VD. In addition, within each magnetic field measurement period, the clock signal is input from the TG 70 to the magnetic field generation control unit 71 depending on the preset frequency. The frequency is set to a frequency at which the generation coil 39 that generates the magnetic field can make a round during each magnetic field measurement period, that is, between two consecutive frame start signals VD. In addition, the frequency is also set in advance in the magnetic field measurement control unit 58, and the generation of the magnetic field from each generation coil 39 and the measurement of the magnetic field by the magnetic field measurement control unit 58 are performed in synchronization with each other. The magnetic field generation control unit 71 switches the generation coil 39 to be driven in response to the clock signal input from the TG 70.

In addition, as shown in Fig. 8, all of the detection coils 25 are sequentially selected by the selector 49C described above within a period in which the magnetic field is generated from one generation coil 39. Note that Fig. 8 shows an example in a case in which the number of detection coils 25 is six. Therefore, the output signal of the magnetic field detection circuit 49 is a signal representing a waveform corresponding to the intensity of the magnetic field detected at a predetermined sampling rate within a selection period in which each of the detection coils 25 is sequentially selected by the selector 49C.

The insertion state detection unit 69 detects the insertion state of the insertion part 17 of the endoscope 10 inserted into the body of the subject H based on the frame start signal VD and the magnetic field measurement data 55 acquired from the image signal acquisition unit 68. The insertion state detection unit 69 generates insertion part shape data 78 indicating the shape of the insertion part 17, and outputs the generated insertion part shape data 78 to the display output unit 74. The insertion state detection unit 69 includes a position detection unit 72 and an insertion part shape detection unit 73. The details of the insertion state detection unit 69 will be described below.

The storage unit 51 stores data used for the correction by the position detection unit 72 of the navigation device 12, which will be described below. The navigation device 12 can acquire the data stored in the storage unit 51 via the light source device 11 and the integrated control circuit 50. Examples of the storage unit 51 include a non-volatile memory. The storage unit 51 is provided on, for example, a substrate provided with the magnetic field detection circuit 49 or the integrated control circuit 50. The substrate is provided inside, for example, the connector 34 of the endoscope 10. Details of the data stored in the storage unit 51 will be described below.

The current detection unit 79 detects the current flowed to the generation coil 39 by the magnetic field generation control unit 71 for driving the generation coil 39 in synchronization with the drive of the generation coil 39 by the magnetic field generation control unit 71. Moreover, the current detection unit 79 outputs the detected current to the insertion state detection unit 69. Therefore, the insertion state detection unit 69 can acquire, from the current detection unit 79, the waveform of the current (hereinafter, referred to as "drive waveform") flowed to the generation coil 39 by the magnetic field generation control unit 71 for driving the generation coil 39.

The display output unit 74 outputs the frame image signal 61 input from the image signal acquisition unit 68 and the insertion part shape data 78 input from the insertion state detection unit 69 to the processor device 14 via communication interfaces 80A and 80B. In this case, the display output unit 74 associates the frame image signal 61 with the insertion part shape data 78 that corresponds in time with the frame image signal 61, and outputs the data to the processor device 14.

<Processor Device>

The processor device 14 has a display input unit 82 and a display control unit 83. The display input unit 82 sequentially outputs, to the display control unit 83, the data of the frame image signal 61 and the insertion part shape data 78, which are sequentially input from the display output unit 74 via the communication interfaces 80A and 80B.

The display control unit 83 receives the input of the frame image signal 61 and the insertion part shape data 78 from the display input unit 82, and displays the observation image 41 (motion picture) based on the frame image signal 61 and the insertion part shape image 42 based on the insertion part shape data 78 on the monitor 15. As described above, the frame image signal 61 is transmitted from the endoscope 10 to the processor device 14 via the light source device 11 and the navigation device 12. Moreover, the processor device 14 performs image processing on the frame image signal 61 acquired from the endoscope 10 to generate the observation image 41 which is an example of a display image.

<Details of Endoscope Insertion State Detection>

By the way, it is possible to derive the intensity of the magnetic field by normalizing the voltage of the electromotive force generated by the detection coil 25 with the differential value of the current for driving the generation coil 39. Specifically, as shown in Fig. 9, the waveform of the magnetic field received by the detection coil 25 and the differential waveform obtained by differentiating the drive waveform of the generation coil 39 are similar, and a degree of similarity represents the intensity of the magnetic field. Therefore, according to the invention, the intensity of the magnetic field is derived by normalizing the magnetic field measurement result of the detection coil 25 with the differential waveform obtained by differentiating the drive waveform of the generation coil 39.

However, the pair of the instrumentation amplifier 49A and the filter 49B connected to each detection coil 25 has different frequency characteristics, and the influence of the frequency characteristics on the magnetic field measurement result is also different. The above is due to variations among individuals. Therefore, in a case in which the magnetic field measurement result is normalized by the differential waveform without considering the frequency characteristic, the intensity of the magnetic field may not be able to be derived with high accuracy. Therefore, in the present embodiment, the frequency characteristics of each pair of the instrumentation amplifier 49A and the filter 49B are stored in the storage unit 51. The frequency characteristic is measured, for example, after the completion of manufacture and before shipping of the endoscope system 9 at the factory, and is stored in the storage unit 51.

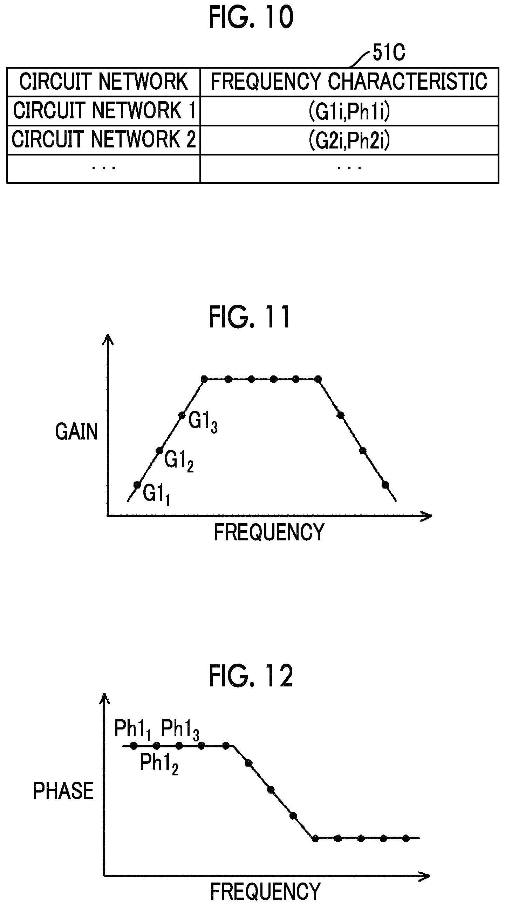

Fig. 10 shows an example of a frequency characteristic table 51C stored in the storage unit 51. As shown in Fig. 10, in the frequency characteristic table 51C, the frequency characteristics of each of a plurality of pairs of the instrumentation amplifier 49A and the filter 49B are stored in association with each pair of the instrumentation amplifier 49A and the filter 49B. "i" (i = 1 to n) in Fig. 10 is a subscript, and the number of data that can reproduce the waveform of the frequency characteristic is stored.

A gain and a phase are stored as the frequency characteristics in the frequency characteristic table 51C. Specifically, as shown in Fig. 11 as an example, the frequency characteristic table 51C stores a predetermined gain for each frequency, as the frequency characteristic. In addition, as shown in Fig. 12 as an example, the frequency characteristic table 51C stores a predetermined phase for each frequency, as the frequency characteristic. In both Figs. 11 and 12, points of the gain and the phase stored in the frequency characteristic table 51C are indicated by circles.

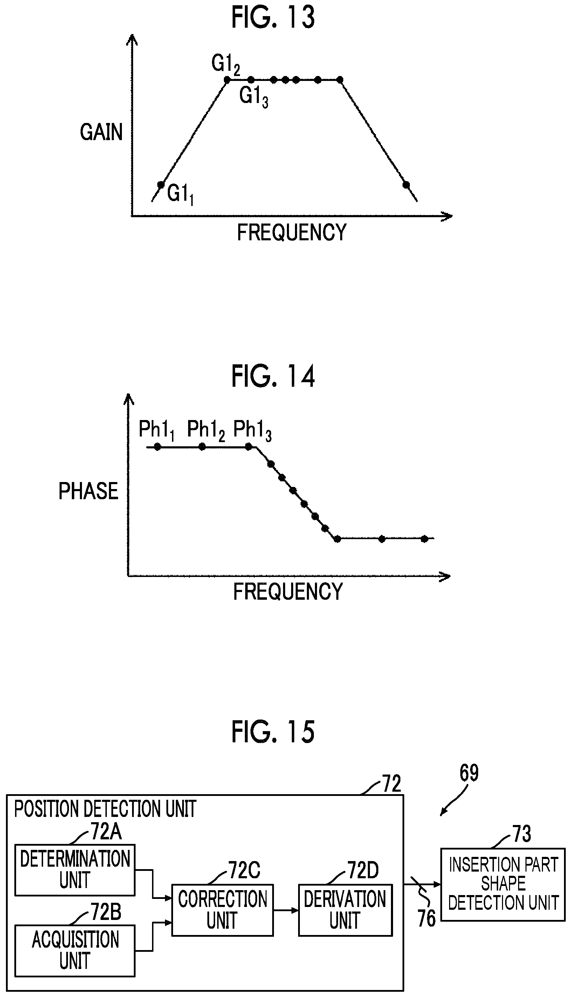

Note that, for the gain and the phase stored in the frequency characteristic table 51C, as shown in Figs. 13 and 14 as an example, the number of points to be stored may be increased in the frequency band in which the waveform reproducibility is desired to be improved, and the number of points to be stored may be decreased in other frequency bands. As a result, it is possible to reproduce a desired waveform even with the same storage capacity. In addition, in a case in which the frequency band for which the reproducibility is desired is relatively narrow, it is also possible to reduce the storage capacity.

Next, the details of the insertion state detection unit 69 according to the present embodiment will be described with reference to Fig. 15. As shown in Fig. 15, the insertion state detection unit 69 includes a position detection unit 72 and an insertion part shape detection unit 73. In addition, the position detection unit 72 includes a determination unit 72A, an acquisition unit 72B, a correction unit 72C, and a derivation unit 72D.

As shown in Fig. 7, the determination unit 72A determines the plurality of magnetic field measurement results included in the magnetic field measurement data 55 with reference to a correspondence relationship 75. The correspondence relationship 75 is information indicating a storage order of the plurality of magnetic field measurement results corresponding to a plurality of combinations of the generation coils 39 and the detection coils 25 included in the magnetic field measurement data 55. The determination unit 72A determines which combination of each generation coil 39 and each detection coil 25 corresponds to each magnetic field measurement result included in the magnetic field measurement data 55 based on the correspondence relationship 75.

Specifically, in the magnetic field measurement, a generation order in which the generation coils 39 generate the magnetic field MF with the frame start signal VD as a reference and an acquisition order of the magnetic field measurement results of the detection coils 25 for the magnetic field MF of one generation coil 39 are decided for each magnetic field measurement period. The plurality of magnetic field measurement results corresponding to the combinations of the generation coils 39 and the detection coils 25 are stored in the magnetic field measurement data 55 depending on the generation order and the acquisition order. Therefore, the determination unit 72A can determine which combination of the plurality of magnetic field measurement results included in the magnetic field measurement data 55 ("D11", "D12", "D13", ...) corresponds to each magnetic field measurement result by referring to the correspondence relationship 75 that defines the storage order with the frame start signal VD as a reference.

The acquisition unit 72B acquires the drive waveform of the generation coil 39 detected by the current detection unit 79. In addition, the acquisition unit 72B acquires the frequency characteristic of each pair of the instrumentation amplifier 49A and the filter 49B from the frequency characteristic table 51C via the light source device 11 and the integrated control circuit 50.

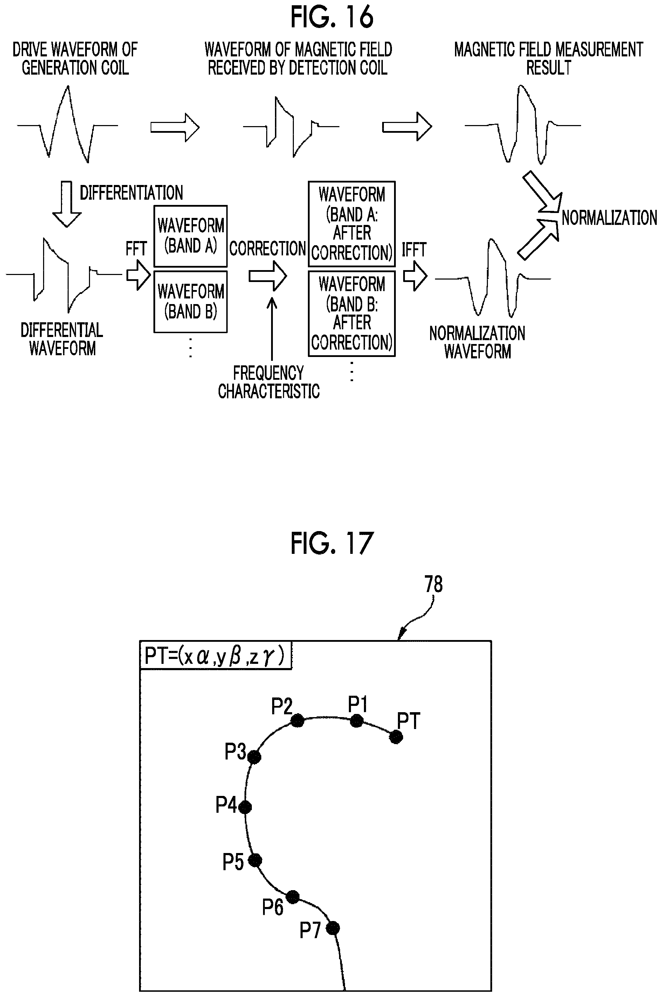

The details of the correction unit 72C will be described with reference to Fig. 16. The correction unit 72C differentiates the drive waveform of the generation coil 39 acquired by the acquisition unit 72B. As a result, as shown in Fig. 16, the differential waveform is obtained. Next, the correction unit 72C separates the differential waveform into a plurality of components of different frequency bands by performing a fast Fourier transform (FFT) process on the differential waveform. Next, the correction unit 72C corrects the amplitude of the component of each separated frequency band depending on the gain of the pair of the instrumentation amplifier 49A and the filter 49B acquired by the acquisition unit 72B. In addition, the correction unit 72C corrects the phase of each separated frequency band component depending on the phase of the pair of the instrumentation amplifier 49A and the filter 49B acquired by the acquisition unit 72B.

Next, the correction unit 72C synthesizes the corrected component of each frequency band by performing an inverse fast Fourier transform (IFFT) process. The correction unit 72C generates the normalization waveform used for the normalization by the synthesis. The correction unit 72C performs the process described above individually for each pair of the instrumentation amplifier 49A and the filter 49B. Therefore, the normalization waveform that has been corrected depending on each frequency characteristic can be obtained corresponding to each pair of the instrumentation amplifier 49A and the filter 49B, that is, each detection coil 25.

The derivation unit 72D normalizes each magnetic field measurement result determined by the determination unit 72A by the normalization waveform generated by the correction unit 72C corresponding to each magnetic field measurement result. As a result, the derivation unit 72D derives the intensity of the magnetic field corresponding to each magnetic field measurement result. Specifically, the derivation unit 72D performs the normalization by dividing the magnetic field measurement result by the normalization waveform. In the present embodiment, since the normalization waveform is generated by correcting the differential waveform depending on the frequency characteristic, the intensity of the magnetic field can be derived with high accuracy.

The position detection unit 72 detects, as coil position data 76, the position of each detection coil 25, specifically, the three-dimension coordinate position based on the intensity of the plurality of magnetic fields determined by the determination unit 72A and derived by the derivation unit 72D. The coil position data 76 is a relative position with the magnetic field generator 13 as a reference. In Fig. 7, for example, P1 indicates the three-dimension coordinate position $(x1,y1,z1)$ of the first detection coil 25. The same applies to P2, P3, P4, and the like.

The position detection unit 72 outputs the coil position data 76 to the insertion part shape detection unit 73. The insertion part shape detection unit 73 detects the shape of the insertion part 17 in the body of the subject H based on the coil position data 76 input from the position detection unit 72.

Fig. 17 is an explanatory diagram for describing an example of a shape detection process of the insertion part 17 by the insertion part shape detection unit 73. As shown in Fig. 17, the insertion part shape detection unit 73 performs an interpolation process of performing interpolation on each position with a curve based on the position (P1, P2, ...) of each detection coil 25 indicated by the coil position data 76, and generates insertion part shape data 78 showing the shape of the insertion part 17. The insertion part shape data 78 includes a distal end position PT of the distal end part 23 of the insertion part 17.

The insertion part shape detection unit 73 outputs the insertion part shape data 78 to the display output unit 74. Each time the image signal acquisition unit 68 acquires a new frame image signal 61, the insertion state detection unit 69 repeatedly performs a position detection process by the position detection unit 72, the shape detection process by the insertion part 17, and a process of outputting the insertion part shape data 78.

<Operation of Endoscope System>

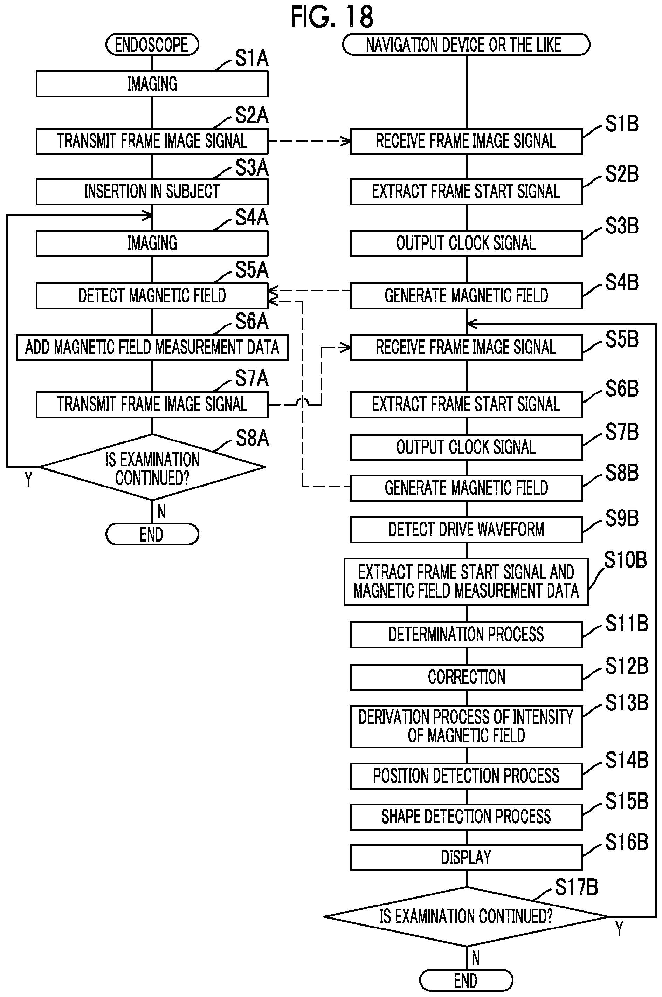

Next, an operation of the endoscope system 9 according to the present embodiment will be described with reference to Fig. 18. Note that Fig. 18 is a flowchart showing an example of a display process of the observation image 41 and the insertion part shape image 42. The display process is executed, for example, after the power switch of the endoscope system 9 is turned on and each unit of the endoscope system 9 is activated. None of the navigation or movement steps disclosed, herein, forms part of the claimed invention.

In step S1A of Fig. 18, the imaging element 53 of the imaging apparatus 48 images the image light incident through the observation window 47 and the condenser lens 52. As a result, the imaging element 53 outputs the image signal to the integrated control circuit 50 with reference to the reference signal oscillated from the oscillation unit 53a. The image signal input from the imaging element 53 to the integrated control circuit 50 is output to the image signal output unit 59 as the frame image signal 61 after various signal processes are performed by the signal processing unit 57 of the integrated control circuit 50. The frame image signal 61 input from the signal processing unit 57 to the image signal output unit 59 is output to the LD 36 after the image signal output unit 59 adds the frame start signal VD.

In step S2A, the LD 36 transmits the optical signal obtained by converting the frame image signal 61 input from the image signal output unit 59 into the optical signal to the PD 37 of the light source device 11.

In step S1B, the PD 37 of the light source device 11 receives the optical signal transmitted from the LD 36. In addition, the PD 37 converts the frame image signal 61 received in a form of the optical signal into a form of the original electric signal. The converted frame image signal 61 from the PD 37 is output to the navigation device 12 via the light source control unit 64 and the communication interface 65.

In step S2B, the image signal acquisition unit 68 acquires the frame image signal 61 from the light source control unit 64 via the communication interface 65 and extracts the frame start signal VD from the acquired frame image signal 61. Moreover, the image signal acquisition unit 68 outputs the extracted frame start signal VD to the TG 70.

In step S3B, the TG 70 outputs the clock signal for control of switching each generation coil 39 to the magnetic field generation control unit 71 based on the frame start signal VD from the image signal acquisition unit 68. In step S4B, the magnetic field generation control unit 71 drives the generation coils 39 at different timings based on the clock signal input from the TG 70. As a result, the magnetic fields are generated from the generation coils 39 at different timings. By the processes up to step S4B described above, the activation of the endoscope system 9 is completed.

Next, in step S3A, that does not form part of the claimed invention, the insertion part 17 of the endoscope 10 is inserted into the subject H by the operator OP, and imaging of the observation site in the subject H is started. The illumination light supplied from the illumination light source 63 of the light source device 11 is emitted from the illumination window 46 to the observation site through the light guide 33 and the irradiation lens 45. In step S4A, the imaging element 53 images the image light of the observation site incident through the observation window 47 and the condenser lens 52. As a result, the imaging element 53 outputs the image signal to the integrated control circuit 50 with reference to the reference signal oscillated from the oscillation unit 53a. The image signal input from the imaging element 53 to the integrated control circuit 50 is output to the image signal output unit 59 as the frame image signal 61 after various signal processes are performed by the signal processing unit 57 of the integrated control circuit 50.

In step S5A, the magnetic field measurement control unit 58 controls the magnetic field detection circuit 49 at the frequency corresponding to the clock signal of the TG 70 based on the reference signal oscillated from the oscillation unit 53a, and repeatedly acquires the magnetic field measurement results detected by the detection coils 25. That is, the magnetic field measurement control unit 58 acquires the magnetic field measurement result, for each generation coil 39, detected by each detection coil 25 in synchronization with the switching of the generation coil 39. Moreover, the magnetic field measurement control unit 58 outputs the magnetic field measurement data 55 including all of the acquired magnetic field measurement results to the image signal output unit 59 in synchronization with the output of the frame image signal 61 from the signal processing unit 57 to the image signal output unit 59.

In step S6A, the image signal output unit 59 adds the frame start signal VD to the frame image signal 61 input from the signal processing unit 57, and adds the magnetic field measurement data 55 input from the magnetic field measurement control unit 58 to the signal invalid region ND. In step S7A, the image signal output unit 59 outputs, to the LD 36, the frame image signal 61 to which the frame start signal VD and the magnetic field measurement data 55 are added. The LD 36 transmits the optical signal obtained by converting the frame image signal 61 input from the image signal output unit 59 into the optical signal to the PD 37 of the light source device 11.

In step S5B to step S8B, the same processes as in step S1B to step S4B are executed.

In step S9B, as described above, the current detection unit 79 detects the drive waveform of the generation coil 39. In step S10B, the image signal acquisition unit 68 extracts the frame start signal VD and the magnetic field measurement data 55 included in the frame image signal 61 acquired in step S6B, and outputs the extracted frame start signal VD and the magnetic field measurement data 55 to the insertion state detection unit 69.

In step S11B, as described above, the determination unit 72A determines the plurality of magnetic field measurement results included in the magnetic field measurement data 55 input from the frame image signal 61 with reference to the correspondence relationship 75.

In step S12B, the acquisition unit 72B acquires the frequency characteristic of each pair of the instrumentation amplifier 49A and the filter 49B from the frequency characteristic table 51C via the light source device 11 and the integrated control circuit 50. The correction unit 72C differentiates the drive waveform of the generation coil 39 detected in step S9B. In addition, as described above, the correction unit 72C generates the normalization waveform by correcting the differential waveform obtained by differentiating the drive waveform depending on the frequency characteristic acquired by the acquisition unit 72B.

In step S13B, as described above, the derivation unit 72D derives the intensity of the magnetic field corresponding to each magnetic field measurement result based on each magnetic field measurement result determined in step S11B and the normalization waveform generated in step S12B.

In step S14B, as described above, the position detection unit 72 detects the coil position data 76 based on the intensity of the plurality of magnetic fields determined in step S11B and derived in step S13B. Moreover, the position detection unit 72 outputs the detected coil position data 76 to the insertion part shape detection unit 73.

In step S15B, as described above, the insertion part shape detection unit 73 detects the shape of the insertion part 17 in the body of the subject H based on the coil position data 76 input from the position detection unit 72, and generates the insertion part shape data 78 indicating the shape of the insertion part 17.

In step S16B, the display output unit 74 outputs the frame image signal 61 acquired by the image signal acquisition unit 68 in step S6B and the insertion part shape data 78 generated by the insertion part shape detection unit 73 in step S15B to the processor device 14 via the communication interfaces 80A and 80B. The frame image signal 61 and the insertion part shape data 78, which are input from the display output unit 74 to the display input unit 82 of the processor device 14, are output to the monitor 15 by the display control unit 83. As a result, the observation image 41 based on the frame image signal 61 and the insertion part shape image 42 based on the insertion part shape data 78 are displayed on the monitor 15.

The processes of step S4A to step S7A and the processes of step S5B to step S16B described above are repeatedly performed until the endoscopic examination is completed (steps S8A and S17B). As a result, the observation image 41 and the insertion part shape image 42 are updated and displayed on the monitor 15 depending on the preset frame rate.

As described above, according to the present embodiment, the normalization waveform is generated by correcting the differential waveform obtained by differentiating the drive waveform of the generation coil 39 depending on the frequency characteristic of the pair of the instrumentation amplifier 49A and the filter 49B. Therefore, by normalizing the magnetic field measurement result with the normalization waveform, the intensity of the magnetic field can be derived with high accuracy, and as a result, the insertion state of the insertion part of the endoscope can be detected with high accuracy.

In addition, according to the present embodiment, the frequency characteristic table 51C is stored in the storage unit 51 provided in the endoscope 10. Therefore, even in a case in which only the endoscope 10 of the endoscope system 9 is replaced, the insertion state of the insertion part of the endoscope can be detected with high accuracy by the same process as in the first embodiment without modifying the program of the navigation device 12.

[Second Embodiment]

A second embodiment of the disclosed technology will be described. In the present embodiment, the same parts as those in the first embodiment are added by the same reference numerals, the description thereof will be omitted, and only the different parts will be described.

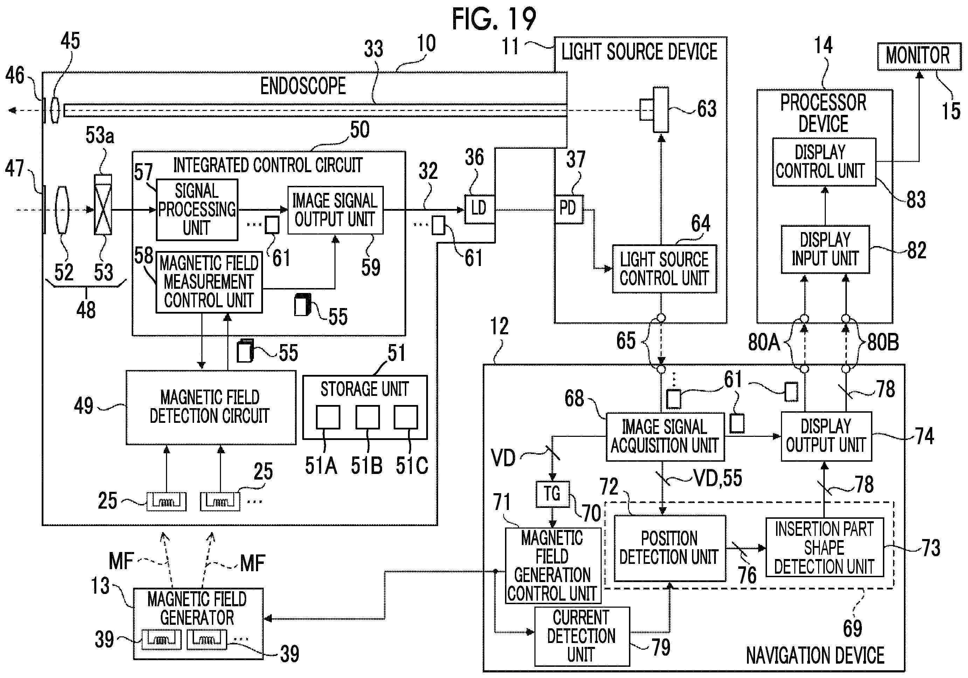

Each detection coil 25 has variations among individuals, and depending on the combination of the detection coil 25 and the instrumentation amplifier 49A connected to each other, the influences on the magnetic field measurement result based on the detection signal of the detection coil 25 are different. Therefore, in the endoscope system 9 according to the present embodiment, as shown in Fig. 19, in addition to the frequency characteristic table 51C, a resistance value table 51A and a capacitance table 51B are stored in the storage unit 51 provided in the endoscope 10. In the resistance value table 51A, a value relating to output impedance of each detection coil 25 is stored in association with each detection coil 25. In the capacitance table 51B, a value relating to input impedance of each instrumentation amplifier 49A is stored in association with each instrumentation amplifier 49A.

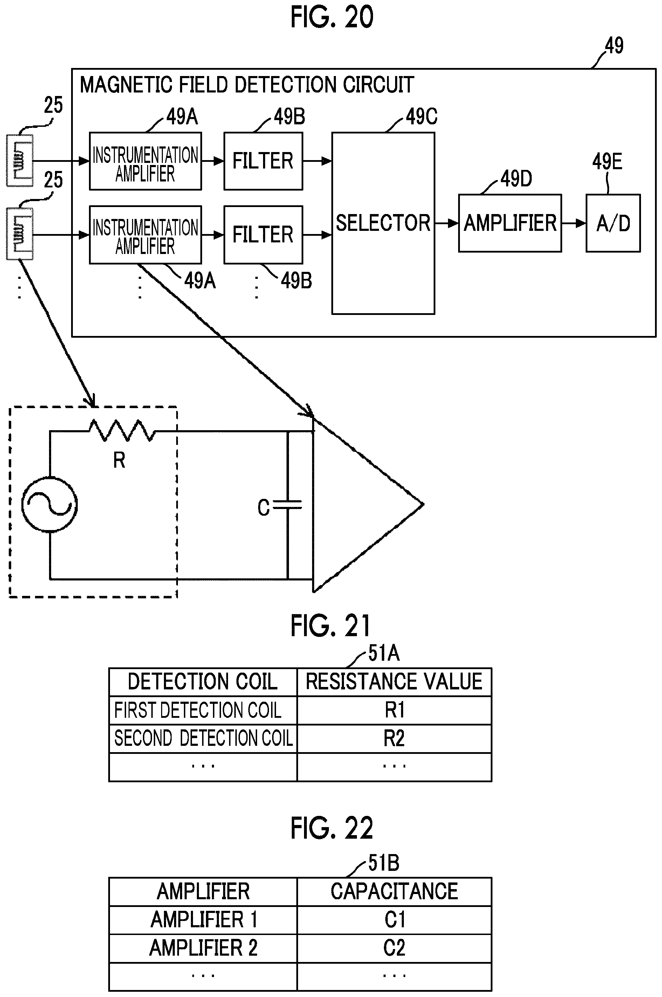

Fig. 20 shows an example of an equivalent circuit of the detection coil 25 and a circuit configuration of the instrumentation amplifier 49A according to the present embodiment. As shown in Fig. 20, the detection coil 25 is represented by a power supply and a resistor R. In addition, a capacitor C is provided in a front stage of the instrumentation amplifier 49A. The LPF is configured by a combination of the resistor R and the capacitor C. In the present embodiment, a resistance value of the detection coil 25 is stored in the storage unit 51 as the value relating to the output impedance of the detection coil 25. In addition, capacitance of the capacitor C is stored in the storage unit 51 as the value relating to the input impedance of the instrumentation amplifier 49A. The resistance value of each detection coil 25 and the capacitance of each capacitor C are measured, for example, after the completion of manufacture and before shipping of the endoscope system 9 at the factory, and are stored in the storage unit 51.

Fig. 21 shows an example of the resistance value table 51A stored in the storage unit 51. As shown in Fig. 21, in the resistance value table 51A, the resistance value of each of the plurality of detection coils 25 is stored in association with each detection coil 25.

Fig. 22 shows an example of the capacitance table 51B stored in the storage unit 51. As shown in Fig. 22, in the capacitance table 51B, the capacitance of the capacitor C corresponding to each of the plurality of instrumentation amplifiers 49A is stored in association with each instrumentation amplifier 49A. Note that the resistance value table 51A and the capacitance table 51B may be stored in separate storage units.

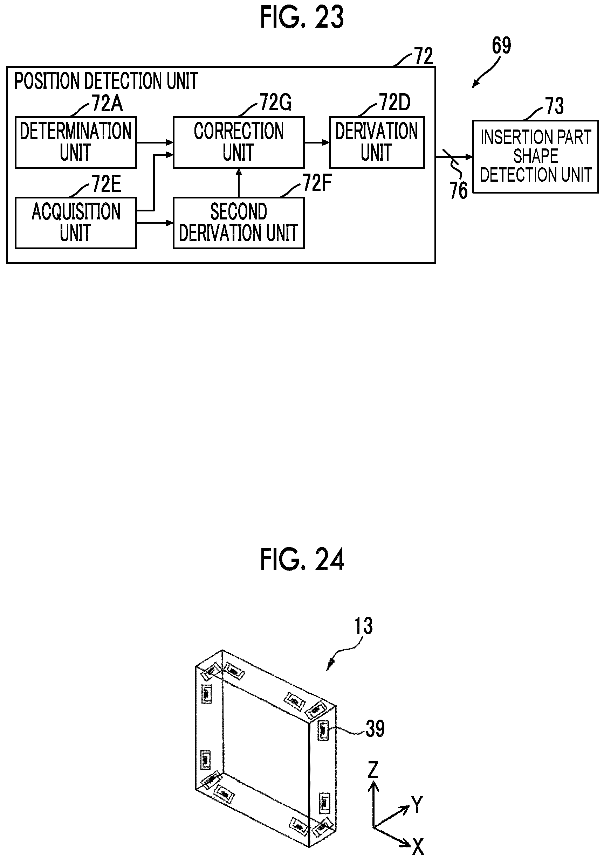

Next, the details of the insertion state detection unit 69 according to the present embodiment will be described with reference to Fig. 23. As shown in Fig. 23, the insertion state detection unit 69 includes a position detection unit 72 and an insertion part shape detection unit 73. In addition, the position detection unit 72 includes the determination unit 72A, an acquisition unit 72E, a second derivation unit 72F, a correction unit 72G, and a derivation unit 72D.

The acquisition unit 72E has the following functions in addition to the function of the acquisition unit 72E according to the first embodiment. The acquisition unit 72E acquires the resistance value of each detection coil 25 from the resistance value table 51A via the light source device 11 and the integrated control circuit 50. In addition, the acquisition unit 72E acquires the capacitance of the capacitor C corresponding to each instrumentation amplifier 49A connected to each detection coil 25 from the capacitance table 51B via the light source device 11 and the integrated control circuit 50.

The second derivation unit 72F derives the frequency characteristics of the detection coil 25, and the pair of the instrumentation amplifier 49A connected to the detection coil 25 by using the resistance value and the capacitance acquired by the acquisition unit 72E. In the following, for the sake of distinction, the frequency characteristic of the pair of the instrumentation amplifier 49A and the filter 49B stored in the frequency characteristic table 51C is referred to as a "first frequency characteristic", and the frequency characteristic of the pair of the detection coil 25 and the instrumentation amplifier 49A derived by the second derivation unit 72F is referred to as a "second frequency characteristic".

Specifically, the second derivation unit 72F derives the gain for each of the pair of the detection coil 25 and the instrumentation amplifier 49A according to Expression (1) by using the corresponding resistance value and capacitance. In addition, the second derivation unit 72F derives the phase for each of the pair of the detection coil 25 and the instrumentation amplifier 49A according to Expression (2) by using the corresponding resistance value and capacitance. The gain and the phase are examples of the second frequency characteristic. Note that, in Expression (1) and Expression (2), C represents the capacitance, R represents the resistance value, and $\omega$ represents the angular frequency ($\omega = 2\pi \times f$).

$$\text{Gain} = 20 \times \log_{10}\left(\frac{1}{\sqrt{1+(\omega CR)^2}}\right) \quad \cdots \quad (1)$$

$$\text{Phase} = \tan^{-1}(-\omega CR) \quad \cdots \quad (2)$$

The correction unit 72G generates the normalization waveform by correcting the differential waveform depending on the second frequency characteristic derived by the second derivation unit 72F in addition to the first frequency characteristic acquired by the acquisition unit 72E. Specifically, the correction unit 72G differentiates the drive waveform of the generation coil 39 acquired by the acquisition unit 72E. As a result, the differential waveform can be obtained. Next, the correction unit 72G separates the differential waveform into a plurality of components of different frequency bands by performing the FFT process on the differential waveform. Next, the correction unit 72G corrects the amplitude of the component of each separated frequency band depending on the gain acquired by the acquisition unit 72E and the gain derived by the second derivation unit 72F. In addition, the correction unit 72G corrects the phase of the component of each separated frequency band depending on the phase acquired by the acquisition unit 72E and the phase derived by the second derivation unit 72F.

Next, the correction unit 72G synthesizes the corrected component of each frequency band by performing the IFFT process. The correction unit 72G generates the normalization waveform by the synthesis. The correction unit 72G performs the process described above individually for each pair of the detection coil 25, the instrumentation amplifier 49A, and the filter 49B. Therefore, the normalization waveform obtained with correction depending on the first frequency characteristic and the second frequency characteristic corresponding to each detection coil 25 can be obtained. The normalization waveform is used for the derivation process of the intensity of the magnetic field by the derivation unit 72D as in the first embodiment.

Next, an operation of the endoscope system 9 according to the present embodiment will be described with reference to Fig. 18. Note that the operation of the endoscope system 9 according to the present embodiment is different from the operation according to the first embodiment in the process of step S12B of Fig. 18, and thus only the process of step S12B will be described.

In step S12B of Fig. 18, the acquisition unit 72E acquires the first frequency characteristic of each pair of the instrumentation amplifier 49A and the filter 49B from the frequency characteristic table 51C via the light source device 11 and the integrated control circuit 50. In addition, the acquisition unit 72E acquires the resistance value of each detection coil 25 from the resistance value table 51A via the light source device 11 and the integrated control circuit 50. In addition, the acquisition unit 72E acquires the capacitance of the capacitor C corresponding to each instrumentation amplifier 49A connected to each detection coil 25 from the capacitance table 51B via the light source device 11 and the integrated control circuit 50. The second derivation unit 72F derives the second frequency characteristic of each pair of the detection coil 25 and the instrumentation amplifier 49A according to Expression (1) and Expression (2) by using the resistance value and capacitance acquired by the acquisition unit 72E. The correction unit 72C differentiates the drive waveform of the generation coil 39 detected in step S9B. In addition, as described above, the correction unit 72C generates the normalization waveform by correcting the differential waveform obtained by differentiating the drive waveform depending on the first frequency characteristic acquired by the acquisition unit 72E and the second frequency characteristic derived by the second derivation unit 72F.

As described above, according to the present embodiment, it is possible to obtain the same effect as that of the first embodiment. In addition, according to the present embodiment, the normalization waveform is generated by correcting the differential waveform obtained by differentiating the drive waveform of the generation coil 39 depending on the second frequency characteristic of each pair of the detection coil 25 and the instrumentation amplifier 49A in addition to the first frequency characteristic of each pair of the instrumentation amplifier 49A and the filter 49B. Therefore, by normalizing the magnetic field measurement result with the normalization waveform, the intensity of the magnetic field can be derived with higher accuracy, and as a result, the insertion state of the insertion part of the endoscope can be detected with higher accuracy.

Note that, in each of the embodiments described above, the example has been described in which the magnetic field measurement unit including the magnetic field measurement control unit 58 is disposed in the endoscope 10 and the magnetic field generation control unit 71 is disposed in the navigation device 12, on the contrary, the magnetic field generation control unit 71 may be disposed in the endoscope 10, and the magnetic field measurement unit may be disposed in the navigation device 12.

In addition, in each of the embodiments described above, the case has been described in which the frame start signal VD included in the frame image signal 61 is used as the synchronization signal. However, a signal other than the signal included in the vertical blanking period, for example, a horizontal synchronization signal and the like may be used as the synchronization signal. In addition, the synchronization signal may be embedded in header information of the frame image signal 61 and used. Further, the signal different from the signal included in the frame image signal 61 may be used as the synchronization signal. In this case, a form is adopted in which a transmission route different from that of the frame image signal 61 is provided.

In addition, in each of the embodiments described above, the case has been described in which the total magnetic field measurement data including the magnetic field measurement results relating to all of the combinations of each generation coil 39 and each detection coil 25 is acquired between two consecutive frame start signals VD, but the present disclosure is not limited to this. For example, total magnetic field measurement data may be acquired in a period in which three or more frame start signals VD are output. The update frequency of the insertion part shape image 42 is decreased as the acquisition period of the total magnetic field measurement data is longer. However, since the insertion part shape image 42 is not required to have a high definition image as compared with the observation image 41, it is permissible in a case of the insertion part shape image 42 even in a case in which the update frequency is decreased as compared with the observation image 41.

In addition, in each of the embodiments described above, the case has been described in which the frame image signal 61 is converted into the optical signal and transmitted from the endoscope 10 to the light source device 11, but the present disclosure is not limited to this. For example, a form may be adopted in which the endoscope 10 and the light source device 11 are electrically connected via the metal signal line, and the frame image signal 61 is transmitted from the endoscope 10 to the light source device 11 as the electric signal.

In addition, in each of the embodiments described above, the endoscope 10 used for the examination of the lower digestive tract, such as the large intestine, has been described as an example, but the type and use of the endoscope 10 are not particularly limited. The endoscope 10 for the upper digestive tract may be used. In addition, particularly in a case in which the purpose is to detect the distal end position PT of the insertion part 17, the disclosed technology can be applied to a soft endoscope as well as a rigid endoscope.

In addition, in each of the embodiments described above, the example has been described in which each generation coil 39 generates the magnetic field MF having the same frequency, but the frequencies of the magnetic fields MF generated by the generation coils 39 may be different.

In addition, in each of the embodiments described above, the plurality of generation coils 39 and the plurality of detection coils 25 are provided, but at least one detection coil 25 need only be provided. For example, the technology of the present disclosure may be applied to the endoscope system 9 using the endoscope 10 in which only one detection coil 25 is provided at the distal end part 23 of the insertion part 17. Even in this case, it is possible to present the current position of the distal end part 23.

In addition, in each of the embodiments described above, each magnetic field measurement result included in the magnetic field measurement data 55 may be corrected depending on the actual installation position and the installation angle of the generation coil 39 in the housing of the magnetic field generator 13. As shown in Fig. 24, as an example, a case will be described in which the housing of the magnetic field generator 13 is a rectangular parallelepiped and each side of the rectangular parallelepiped is the X-axis, the Y-axis, and the Z-axis. Here, it is assumed that each generation coil 39, which is a three-axis coil, is disposed at the installation angle along the X-axis, the Y-axis, and the Z-axis, and at a predetermined installation position. In this case, the coil position data 76 generated by the position detection unit 72 of the navigation device 12 is premised on the installation position and installation angle of each generation coil 39 being predetermined installation position and installation angle. However, the installation position and the installation angle of each generation coil 39 may deviate from the predetermined installation position and installation angle due to an error during manufacture or the like.

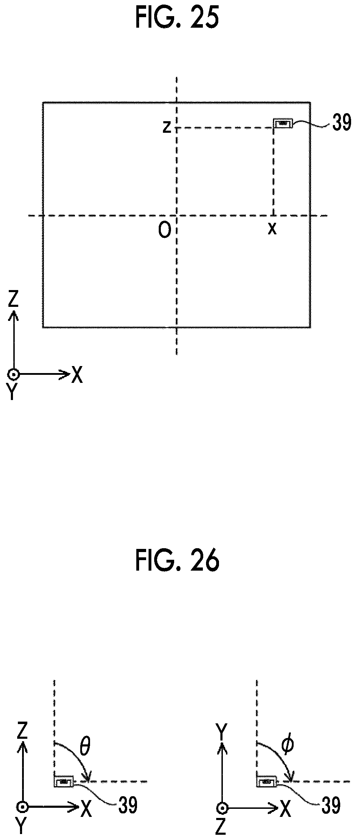

Therefore, the actual installation position and installation angle of each generation coil 39 are measured after the magnetic field generator 13 is manufactured and before shipment thereof, and the measured actual installation position and installation angle are stored in, for example, the storage unit, such as a non-volatile memory, provided in the magnetic field generator 13. The navigation device 12 acquires the actual installation position and installation angle of each generation coil 39 from the storage unit of the magnetic field generator 13, and corrects each magnetic field measurement result included in the magnetic field measurement data 55 depending on deviation amounts of the acquired installation position and installation angle with respect to the predetermined installation position and installation angle. Note that, as shown in Fig. 25 as an example, examples of the actual installation position of the generation coil 39 in this case include the XYZ coordinate with a predetermined position (for example, a center position of the housing of the magnetic field generator 13) as the origin. In addition, as shown in Fig. 26 as an example, examples of the actual installation angle of the generation coil 39 in this case include a zenith angle $\theta$ and an azimuthal angle $\varphi$.

In addition, in each of the embodiments described above, for example, as a hardware structure of the processing unit that executes various processes, such as the magnetic field measurement control unit 58 and the magnetic field generation control unit 71, various processors in the following can be used. The various processors include the CPU that is a general-purpose processor executing the software and functioning as the various processing units, as well as a programmable logic device (PLD) that is a processor of which a circuit configuration can be changed after manufacture, such as a field programmable gate array (FPGA) or a dedicated electric circuit that is a processor having a circuit configuration that is designed for exclusive use in order to execute a specific process, such as an application specific integrated circuit (ASIC).

One processing unit may be configured by one of the various processors, or may be a combination of two or more processors of the same type or different types (for example, a combination of a plurality of FPGAs or a combination of a CPU and an FPGA). In this way, as the hardware structure, various processing units are configured by one or more of various processors described above. Stated another way, these processors function as each processing unit in cooperation with the memory built in the processor or the connected memory.

Further, as the hardware structure of these various processors, more specifically, it is possible to use an electric circuit (circuitry) in which circuit elements such as semiconductor elements are combined.

**Claims**

1.   An endoscope system (9) comprising:

> an endoscope (10) having an insertion part (17) configured to be inserted into the body of a subject (H),
> an acquisition unit (72B) configured to acquire a drive waveform of a magnetic field generation element generating a magnetic field and a frequency characteristic of a circuit network, said circuit network being connected to a magnetic field detection element (25) configured to detect a magnetic field and said circuit network being configured to output a detection signal corresponding to the magnetic field detected by the magnetic field detection element, (25) the frequency characteristic including a gain and a phase of the circuit network;
> a correction unit (72C) that is configured to generate a normalization waveform by correcting, depending on the frequency characteristic, a differential waveform, the correction unit being configured to obtain the differential waveform by time differentiating the drive waveform wherein the correcting comprises separating the differential waveform into a plurality of different frequency bands by performing a fast Fourier transform, FFT, process on the differential waveform, correcting the amplitude of the component of each separated frequency band depending on the gain of the frequency characteristic and correcting the phase of each separated frequency band depending on the phase of the frequency characteristic, and synthesizing the corrected component of each frequency band by performing an inverse fast Fourier transform, IFFT, process;
> a derivation unit (72D) configured to derive the intensity of the magnetic field by normalizing the detection signal output from the circuit network with the normalization waveform by dividing the detected magnetic field by the normalization waveform; and
an insertion state detection unit (69) configured to detect an insertion state including at least one of a position or a shape of the insertion part (17) of the endoscope (10) inserted into the subject by using the intensity of the magnetic field, wherein the magnetic field detection element (25) is provided in the insertion part (17) of the endoscope (10).

2. The endoscope system according to claim 1,
wherein the frequency characteristic is stored in a storage unit provided in the endoscope.

3. The endoscope system according to claim 1 or 2, further comprising:

a second derivation unit (72F) that is configured to derive, by using a value relating to output impedance of the magnetic field detection element and a value relating to input impedance of a reception circuit provided in the circuit network and connected to the magnetic field detection element, a frequency characteristic of a pair of the magnetic field detection element and the reception circuit,
wherein the correction unit is configured to generate the normalization waveform by correcting the differential waveform depending on a second frequency characteristic derived by the second derivation unit in addition to a first frequency characteristic acquired by the acquisition unit.

4. The endoscope system according to claim 3,

wherein the value relating to the output impedance is a resistance value of the magnetic field detection element, and
the value relating to the input impedance is capacitance of a capacitor provided in a front stage of the reception circuit.

5. An operation method of an endoscope system comprising:

an acquisition step of acquiring, using an acquisition unit, a drive waveform of a magnetic field generation element generating a magnetic field, and a frequency characteristic of a circuit network, which is connected to a magnetic field detection element detecting a magnetic field and which outputs a detection signal corresponding to the magnetic field detected by the magnetic field detection element, the frequency characteristic including a gain and a phase of the circuit network;
a correction step of generating, using a correction unit, a normalization waveform by correcting a differential waveform, which is obtained by time differentiating the drive waveform, depending on the frequency characteristic, wherein the correcting comprises separating the differential waveform into a plurality of different frequency bands by performing a fast Fourier transform, FFT, process on the differential waveform, correcting the amplitude of the component of each separated frequency band depending on the gain of the frequency characteristic and correcting the phase of each separated frequency ban depending on the phase of the frequency characteristic, and synthesizing the corrected component of each frequency band by performing an inverse fast Fourier transform, IFFT, process;
a derivation step of deriving, using a derivation unit, the intensity of the magnetic field detected by the magnetic field detection element by normalizing the detection signal output from the circuit network with the normalization waveform by dividing the detected magnetic field by the normalization waveform; and
an insertion state detection step of detecting, using an insertion state unit, an insertion state including at least one of a position or a shape of an insertion part of an endoscope inserted into a subject by using the intensity of the magnetic field.

**Patentansprüche**

1. Endoskopsystem (9), umfassend:

ein Endoskop (10), das einen Einführteil (17), der so konfiguriert ist, dass er in den Körper einer Untersuchungsperson (H) eingeführt wird, aufweist;
eine Erfassungseinheit (72B), die so konfiguriert ist, dass sie eine Antriebswellenform eines Erzeugungselements für Magnetfeld, das ein Magnetfeld erzeugt, und eine Frequenzeigenschaft eines Schaltungsnetzwerks, wobei das Schaltungsnetzwerk mit einem Magnetfeld-Detektionselement (25), das so konfiguriert ist, dass es ein Magnetfeld detektiert, verbunden ist, und das Schaltungsnetzwerk so konfiguriert ist, dass es ein Detektionssignal, das dem von dem Magnetfeld-Detektionselement (25) detektierten Magnetfeld entspricht, ausgibt, erfasst, wobei die Frequenzeigenschaft eine Verstärkung und eine Phase des Schaltungsnetzwerks umfasst;

eine Korrektureinheit (72C), die so konfiguriert ist, dass sie eine Normalisierungswellenform durch Korrigieren, in Abhängigkeit von der Frequenzeigenschaft, einer Differentialwellenform erzeugt, wobei die Korrektureinheit so konfiguriert ist, dass sie die Differentialwellenform durch zeitliches Differenzieren der Antriebswellenform erhält, wobei das Korrigieren Trennen der Differentialwellenform in mehrere unterschiedliche Frequenzbänder durch Durchführen eines schnellen Fourier-Transformationsprozesses, FFT(fast Fourier transform)-Prozesses, an der Differentialwellenform, das Korrigieren der Amplitude der Komponente jedes getrennten Frequenzbandes in Abhängigkeit von der Verstärkung der Frequenzeigenschaft und das Korrigieren der Phase jedes getrennten Frequenzbandes in Abhängigkeit von der Phase der Frequenzeigenschaft und das Synthetisieren der korrigierten Komponente jedes Frequenzbandes durch Durchführen eines inversen schnellen Fourier-Transformationsprozesses, IFFT(inverse fast Fourier transform)-Prozesses, umfasst;

eine Ableitungseinheit (72D), die so konfiguriert ist, dass sie die Intensität des Magnetfelds durch Normalisieren des von dem Schaltungsnetzwerk ausgegebenen Detektionssignals mit der Normalisierungswellenform durch Teilen des detektierten Magnetfelds durch die Normalisierungswellenform ableitet; und

eine Einführzustands-Detektionseinheit (69), die so konfiguriert ist, dass sie einen Einführzustand, der mindestens eine von einer Position und einer Form des Einführteils (17) des Endoskops (10), der in die Untersuchungsperson eingeführt wird, enthält, durch Verwenden der Intensität des Magnetfelds detektiert, wobei das Magnetfeld-Detektionselement (25) in dem Einführteil (17) des Endoskops (10) vorgesehen ist.

**2.** Endoskopsystem nach Anspruch 1,
wobei die Frequenzeigenschaft in einer Speichereinheit, die in dem Endoskop vorgesehen ist, gespeichert ist.

**3.** Endoskopsystem nach Anspruch 1 oder 2, ferner umfassend:

eine zweite Ableitungseinheit (72F), die so konfiguriert ist, dass sie durch Verwenden eines Werts in Bezug auf Ausgangsimpedanz des Magnetfeld-Detektionselements und eines Werts in Bezug auf Eingangsimpedanz einer Empfangsschaltung, die in dem Schaltungsnetzwerk vorgesehen ist und mit dem Magnetfeld-Detektionselement verbunden ist, eine Frequenzeigenschaft eines Paars des Magnetfeld-Detektionselements und der Empfangsschaltung ableitet,

wobei die Korrektureinheit so konfiguriert ist, dass sie die Normalisierungswellenform durch Korrigieren der Differentialwellenform in Abhängigkeit von einer zweiten Frequenzeigenschaft, die von der zweiten Ableitungseinheit abgeleitet wird, zusätzlich zu einer ersten Frequenzeigenschaft, die von der Erfassungseinheit erfasst wird, erzeugt.

**4.** Endoskopsystem nach Anspruch 3,

wobei der Wert in Bezug auf die Ausgangsimpedanz ein Widerstandswert des Magnetfeld-Detektionselements ist, und

der Wert in Bezug auf die Eingangsimpedanz Kapazität eines Kondensators ist, der in einer Vorstufe der Empfangsschaltung vorgesehen ist.

**5.** Betriebsverfahren eines Endoskopsystems, umfassend:

einen Erfassungsschritt des Erfassens, unter Verwendung einer Erfassungseinheit, einer Antriebswellenform eines Erzeugungselements für Magnetfeld, das ein Magnetfeld erzeugt, und einer Frequenzeigenschaft eines Schaltungsnetzwerks, das mit einem Magnetfeld-Detektionselement, das ein Magnetfeld detektiert, verbunden ist und das ein Detektionssignal, das dem von dem Magnetfeld-Detektionselement detektierten Magnetfeld entspricht, ausgibt, wobei die Frequenzeigenschaft eine Verstärkung und eine Phase des Schaltungsnetzwerks enthält;

einen Korrekturschritt des Erzeugens, unter Verwendung einer Korrektureinheit, einer Normalisierungswellenform durch Korrigieren einer Differentialwellenform, die durch zeitliches Differenzieren der Antriebswellenform erhalten wird, in Abhängigkeit von der Frequenzeigenschaft, wobei das Korrigieren Trennen der Differentialwellenform in mehrere unterschiedliche Frequenzbänder durch Durchführen eines schnellen Fourier-Transformationsprozesses, FFT(fast Fourier transform)-Prozesses, an der Differentialwellenform, das Korrigieren der Amplitude der Komponente jedes getrennten Frequenzbandes in Abhängigkeit von der Verstärkung der Frequenzeigenschaft und das Korrigieren der Phase jedes getrennten Frequenzbandes in Abhängigkeit von der Phase der Frequenzeigenschaft und das Synthetisieren der korrigierten Komponente jedes Frequenzbandes durch Durchführen eines inversen schnellen Fourier-Transformationsprozesses, IFFT(inverse fast Fourier transform)-Prozesses, umfasst;

einen Ableitungsschritt des Ableitens, unter Verwendung einer Ableitungseinheit, der Intensität des Magnetfelds, das von dem Magnetfeld-Detektionselement detektiert wird, durch Normalisieren des von dem Schaltungsnetzwerk ausgegebenen Detektionssignals mit der Normalisierungswellenform durch Teilen des detektierten Magnetfelds durch die Normalisierungswellenform; und

einen Einführzustands-Detektionsschritt des Detektierens, unter Verwendung einer Einführzustandseinheit, eines Einführzustands, der mindestens eine von einer Position und einer Form eines Einführteils eines Endoskops, der in eine Untersuchungsperson eingeführt wird, enthält, durch Verwenden der Intensität des Magnetfelds.

## Revendications

1.  Système d'endoscope (9) comprenant :

    un endoscope (10) comportant une partie d'insertion (17) configurée pour être insérée dans le corps d'un sujet (H) ;

    une unité d'acquisition (72B) configurée pour acquérir une forme d'onde de commande d'un élément de génération de champ magnétique générant un champ magnétique et une caractéristique de fréquence d'un réseau de circuits, ledit réseau de circuits étant connecté à un élément de détection de champ magnétique (25) configuré pour détecter un champ magnétique et ledit réseau de circuits étant configuré pour émettre un signal de détection correspondant au champ magnétique détecté par l'élément de détection de champ magnétique (25), la caractéristique de fréquence incluant un gain et une phase du réseau de circuits ;

    une unité de correction (72C) qui est configurée pour générer une forme d'onde de normalisation en corrigeant, en fonction de la caractéristique de fréquence, une forme d'onde différentielle, l'unité de correction étant configurée pour obtenir la forme d'onde différentielle en différenciant dans le temps la forme d'onde de commande, où la correction comprend la séparation de la forme d'onde différentielle en une pluralité de bandes de fréquence différentes en effectuant un processus de transformation de Fourier rapide, FFT (fast Fourier transform), sur la forme d'onde différentielle, la correction de l'amplitude de la composante de chaque bande de fréquence séparée en fonction du gain de la caractéristique de fréquence et la correction de la phase de chaque bande de fréquence séparée en fonction de la phase de la caractéristique de fréquence, et la synthèse de la composante corrigée de chaque bande de fréquence en effectuant un processus de transformation de Fourier rapide inverse, IFFT (inverse fast Fourier transform) ;

    une unité de dérivation (72D) configurée pour dériver l'intensité du champ magnétique en normalisant le signal de détection émis par le réseau de circuits avec la forme d'onde de normalisation en divisant le champ magnétique détecté par la forme d'onde de normalisation ; et

    une unité de détection d'état d'insertion (69) configurée pour détecter un état d'insertion incluant au moins l'une d'une position ou d'une forme de la partie d'insertion (17) de l'endoscope (10) insérée dans le sujet en utilisant l'intensité du champ magnétique,

    dans lequel l'élément de détection de champ magnétique (25) est prévu dans la partie d'insertion (17) de l'endoscope (10).

2.  Système d'endoscope selon la revendication 1,
    dans lequel la caractéristique de fréquence est stockée dans une unité de stockage prévue dans l'endoscope.

3.  Système d'endoscope selon la revendication 1 ou la revendication 2, comprenant en outre :

    une deuxième unité de dérivation (72F) qui est configurée pour dériver, en utilisant une valeur relative à une impédance de sortie de l'élément de détection de champ magnétique et une valeur relative à une impédance d'entrée d'un circuit de réception prévu dans le réseau de circuits et connecté à l'élément de détection de champ magnétique, une caractéristique de fréquence d'une paire constituée de l'élément de détection de champ magnétique et du circuit de réception,

    dans lequel l'unité de correction est configurée pour générer la forme d'onde de normalisation en corrigeant la forme d'onde différentielle en fonction d'une deuxième caractéristique de fréquence dérivée par la deuxième unité de dérivation en plus d'une première caractéristique de fréquence acquise par l'unité d'acquisition.

4. Système d'endoscope selon la revendication 3,

dans lequel la valeur relative à l'impédance de sortie est une valeur de résistance de l'élément de détection de champ magnétique, et
la valeur relative à l'impédance d'entrée est une capacité d'un condensateur prévu dans un étage avant du circuit de réception.

5. Procédé d'opération d'un système d'endoscope comprenant :

une étape d'acquisition consistant à acquérir, en utilisant une unité d'acquisition, une forme d'onde de commande d'un élément de génération de champ magnétique générant un champ magnétique, et une caractéristique de fréquence d'un réseau de circuits, qui est connecté à un élément de détection de champ magnétique détectant un champ magnétique et qui émet un signal de détection correspondant au champ magnétique détecté par l'élément de détection de champ magnétique, la caractéristique de fréquence incluant un gain et une phase du réseau de circuits ;
une étape de correction consistant à générer, en utilisant une unité de correction, une forme d'onde de normalisation en corrigeant une forme d'onde différentielle, qui est obtenue en différenciant dans le temps la forme d'onde de commande, en fonction de la caractéristique de fréquence, où la correction comprend la séparation de la forme d'onde différentielle en une pluralité de bandes de fréquence différentes en effectuant un processus de transformation de Fourier rapide, FFT (fast Fourier transform), sur la forme d'onde différentielle, la correction de l'amplitude de la composante de chaque bande de fréquence séparée en fonction du gain de la caractéristique de fréquence et la correction de la phase de chaque bande de fréquence séparée en fonction de la phase de la caractéristique de fréquence, et la synthèse de la composante corrigée de chaque bande de fréquence en effectuant un processus de transformation de Fourier rapide inverse, IFFT (inverse fast Fourier transform) ;
une étape de dérivation consistant à dériver, en utilisant une unité de dérivation, l'intensité du champ magnétique détecté par l'élément de détection de champ magnétique en normalisant le signal de détection émis par le réseau de circuits avec la forme d'onde de normalisation en divisant le champ magnétique détecté par la forme d'onde de normalisation ; et
une étape de détection d'état d'insertion consistant à détecter, en utilisant une unité d'état d'insertion, un état d'insertion incluant au moins l'une d'une position ou d'une forme d'une partie d'insertion d'un endoscope insérée dans un sujet en utilisant l'intensité du champ magnétique.

FIG. 1

LIGHT SOURCE DEVICE
PROCESSOR DEVICE
NAVIGATION DEVICE

42
15
41
11
14
12
19
18
10
OP
17
H
9
MF
MF
MF
13
16

FIG. 2

FIG. 3

# FIG. 4

MAGNETIC FIELD DETECTION CIRCUIT — 49

25

49A

INSTRUMENTATION AMPLIFIER

49B

FILTER

49C

SELECTOR

49D

AMPLIFIER

49E

A/D

50

INTEGRATED CONTROL CIRCUIT

25

INSTRUMENTATION AMPLIFIER

FILTER

49A

49B

# FIG. 5

25    25    25    17

FIRST DETECTION COIL

SECOND DETECTION COIL

THIRD DETECTION COIL

...

13

39    39    39

FIRST GENERATION COIL

SECOND GENERATION COIL

THIRD GENERATION COIL

...

MAGNETIC FIELD GENERATOR

# FIG. 6

| | | | | |
|---|---|---|---|---|
| (1) | FIRST DETECTION COIL | SECOND DETECTION COIL | THIRD DETECTION COIL | ... |
| | D11 | D12 | D13 | ... |
| (2) | FIRST DETECTION COIL | SECOND DETECTION COIL | THIRD DETECTION COIL | ... |
| | D21 | D22 | D23 | ... |
| (3) | FIRST DETECTION COIL | SECOND DETECTION COIL | THIRD DETECTION COIL | ... |
| | D31 | D32 | D33 | ... |
| (4) | FIRST DETECTION COIL | SECOND DETECTION COIL | THIRD DETECTION COIL | ... |
| | D41 | D42 | D43 | ... |

55

# FIG. 7

BT

61    61    61    61

VD    VD    VD    VD

FRAME IMAGE SIGNAL | FRAME 1 | ND | FRAME 2 | ND | FRAME 3 | ND | FRAME 4 | ND

55    55    55    55

72A — DETERMINATION UNIT

75 — CORRESPONDENCE RELATIONSHIP

72 — POSITION DETECTION UNIT

76 — COIL POSITION DATA

| | FIRST DETECTION COIL | SECOND DETECTION COIL | THIRD DETECTION COIL | ... |
|---|---|---|---|---|
| FIRST GENERATION COIL | D11 | D12 | D13 | ... |
| SECOND GENERATION COIL | D21 | D22 | D23 | ... |
| THIRD GENERATION COIL | D31 | D32 | D33 | ... |
| FOURTH GENERATION COIL | D41 | D42 | D43 | ... |
| ⋮ | ⋮ | ⋮ | ⋮ | ⋮ |

| COIL POSITION DATA | |
|---|---|
| FIRST DETECTION COIL | P1(x1,y1,z1) |
| SECOND DETECTION COIL | P2(x2,y2,z2) |
| THIRD DETECTION COIL | P3(x3,y3,z3) |
| FOURTH DETECTION COIL | P4(x4,y4,z4) |
| ⋮ | ⋮ |

## FIG. 8

## FIG. 9

DRIVE WAVEFORM OF GENERATION COIL    WAVEFORM OF MAGNETIC FIELD RECEIVED BY DETECTION COIL    MAGNETIC FIELD MEASUREMENT RESULT

DIFFERENTIATION

DIFFERENTIAL WAVEFORM

NORMALIZATION

# FIG. 10

| CIRCUIT NETWORK | FREQUENCY CHARACTERISTIC |
|---|---|
| CIRCUIT NETWORK 1 | $(G1i, Ph1i)$ |
| CIRCUIT NETWORK 2 | $(G2i, Ph2i)$ |
| . . . | . . . |

51C

# FIG. 11

# FIG. 12

## FIG. 13

$G1_2$

$G1_3$

$G1_1$

GAIN

FREQUENCY

## FIG. 14

$Ph1_1$  $Ph1_2$  $Ph1_3$

PHASE

FREQUENCY

## FIG. 15

69

POSITION DETECTION UNIT ⌐72

⌐72A
DETERMINATION
UNIT

⌐72B
ACQUISITION
UNIT

⌐72C
CORRECTION
UNIT

⌐72D
DERIVATION
UNIT

76

⌐73
INSERTION PART
SHAPE
DETECTION UNIT

# FIG. 16

DRIVE WAVEFORM OF
GENERATION COIL

WAVEFORM OF MAGNETIC FIELD
RECEIVED BY DETECTION COIL

MAGNETIC FIELD MEASUREMENT
RESULT

DIFFERENTIATION

FFT

WAVEFORM
(BAND A)

WAVEFORM
(BAND B)

CORRECTION

WAVEFORM
(BAND A:
AFTER
CORRECTION)

WAVEFORM
(BAND B:
AFTER
CORRECTION)

IFFT

NORMALIZATION

DIFFERENTIAL
WAVEFORM

FREQUENCY
CHARACTERISTIC

NORMALIZATION
WAVEFORM

# FIG. 17

78

$PT = (x\alpha, y\beta, z\gamma)$

P2  P1  PT

P3

P4

P5

P6

P7

EP 4 027 183 B1

# FIG. 18

FIG. 19

EP 4 027 183 B1

# FIG. 20

# FIG. 21

| DETECTION COIL | RESISTANCE VALUE |
|---|---|
| FIRST DETECTION COIL | R1 |
| SECOND DETECTION COIL | R2 |
| . . . | . . . |

51A

# FIG. 22

| AMPLIFIER | CAPACITANCE |
|---|---|
| AMPLIFIER 1 | C1 |
| AMPLIFIER 2 | C2 |
| . . . | . . . |

51B

# FIG. 23

# FIG. 24

# FIG. 25

# FIG. 26

**EP 4 027 183 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3432825 B

- EP 3473158 A